# EUROPEAN PATENT APPLICATION

(11) **EP 2 267 447 A1**
(43) Date of publication of application: **29.12.2010**
(21) Application number: 09728954.0
(22) Date of filing: 31.03.2009
(51) Int. Cl.: G01N 33/53, C07K 14/47, C07K 16/18, C12N 15/00, G01N 27/62

(54) **COMPOSITION, KIT AND METHOD FOR DETECTING AGING AND DISEASE ASSOCIATED WITH VASCULAR DISORDER**

(30) Priority: 31.03.2008 JP 2008092245
(71) Applicant: National Hospital Organization, Tokyo 152-8621 (JP); Santen Pharmaceutical Co., Ltd, Osaka-shi Osaka 533-8651 (JP)
(72) Inventor: IWATA, Takeshi, Tokyo 152-8902 (JP); MATSUNO, Kiyoshi, Osaka-shi Osaka 533-8651 (JP); TANAHASHI, Kazuhiro, Otsu-shi Shiga 520-8558 (JP)
(74) Representative: Sutcliffe, Nicholas Robert
(86) International application number: PCT/JP2009/056728
(87) International publication number: WO 2009/123224

(57) **Abstract**

The present invention relates to a method for detecting an ageing or a disease accompanied with a vascular disorder such as age-related macular degeneration, comprising measuring one or more of polypeptides comprising any of amino acid sequences shown in SEQ ID NOS: 1 to 21, mutants thereof, or fragments thereof in a biological sample from a subject, and also to a composition or kit for diagnosing an ageing or a disease accompanied with a vascular disorder such as age-related macular degeneration.

## Description

### TECHNICAL FIELD

The present invention relates to a composition and a kit useful for diagnosis of ageing and a disease accompanied with a vascular disorder.

The present invention also relates to a method for assaying (or determining or identifying) ageing and a disease accompanied with a vascular disorder, using the composition or the kit.

### BACKGROUND ART

Because the average life span has increased due to the progress of medical technology and changes in living and social environments, the elderly population has been continuously increasing in recent years. Acompanied with the increase in elderly population, a variety of diseases affecting the elderly have been highlited. As represented by lifestyle-related diseases, it is said that such diseases develop and progress as a result of the gradual accmulation of small changes occurring in the living body with age. In particular, diseases mainly caused by vascular disorders have become serious social problems.

The "vascular disorder" refers to a condition in which vascular tissue degeneration is induced by a certain cause, triggering bleeding, permeability alteration, aneurysm formation, infarction, angiogenesis, bypass formation, and the like. The development of a vascular disorder causes serious problems in an organ or tissue which is rich in vasculature. If a vascular disorder develops in a sensory organ, and particularly in ocular tissue, it might result in blindness.

The macula is a portion of the retina and is of most concern in relation to vision, and most cells capable of distinguishing colors and shapes are present in this portion. Age-related macular degeneration is a macular disease caused by changes with age. A vascular disorder in ocular tissue is a possible cause of the disease. The disease is a cause of decreased vision among the elderly. Along with a sharp increase in the elderly population in recent years, the number of age-related macular degeneration patients has been continously increasing.

Since the age-related macular degeneration is a disease that results in decreased vision, it is very important to diagnose the disease at an early stage. Hitherto, funduscopy has mainly been performed for the diagnosis of age-related macular degeneration. Prior to the examination, mydriatic eye drops that allow the pupil to dilate are administered to a patient, or otherwise, a fluorescent dye for staining of retinal blood vessels is injected into an arm vein of a patient and subsequently a physician directly observes the retina with a funduscope or fundus camera. However, because the pupils of a patient are kept dilating under the influence of eye drops, the patient remains unable to see due to photophobia for approximately 3 hours. This is stressful for the patient, even if it is temporary. In addition, at present, it cannot be said that there is no risk of causing adverse effects such as nausea, vomiting, and shock after intravenous injection of a fluorescent dye. Moreover, it cannot always be said that direct observation of the retina by a physician is an objective assay method. In direct observation, every patient must be examined by a phisican, and thus it is difficult to apply such examination to mass-screening for the examination of many subjects. Therefore, a high-throughput assay method that is less stressful for patients has been awaited, whereby the degree of pathological progression and time-dependent changes during treatment can be objectively/quantitatively determined for patients.

An assay method using a diagnosis marker is an objective high-throughput method. In the past, a method for diagnosing the age-related macular degeneration using an antibody that specifically recognizes the ED-B domain of fibronectin (Japanese Patent Publication (Kohyo) No. 2002-514405), as well as a method for assaying the same disease using, as a marker, malonate aldehyde in blood plasma (Yildirim O, Ates NA, Tamer L, Muslu N, Ercan B, Atik U, Kanik A., Changes in antioxidant enzyme activity and malondialdehyde level in patients with age-related macular degeneration, Ophthalmologica, 2004 May-Jun, 218(3): 202-206), have been disclosed. It is not possible to determine the age-related macular degeneration with relatively high specificity by the methods using said markers. Thus, at present, such methods remain at a research-stage.

Along with the recent progress in genome analysis (genomics) and proteome analysis (proteomics), a variety of novel marker candidates have been reportedt. For age-related macular degeneration, as a result of the proteome analysis using an ocular tissue, a variety of novel marker candidates have been reported (Ethen CM, Reilly C, Feng X, Olsen TW, Ferrington DA., The proteome of central and peripheral retina with progression of age-related macular degeneration, Invest Ophthalmol Vis Sci. 2006 Jun, 47(6): 2280-2290; and Crabb JW, Miyagi M, Gu X, Shadrach K, West KA, Sakaguchi H, Kamei M, Hasan A, Yan L, Rayborn ME, Salomon RG, Hollyfield JG., Drusen proteome analysis: an approach to the etiology of age-related macular degeneration, Proc Natl Acad Sci U S A. 2002 Nov 12, 99(23): 14682-7). However, there is no report of the discovery of protein markers detected with age-related macular degeneration by proteomics using blood specimens. Also, there is no known method for diagnosing the age-related macular degeneration using protein markers in bloods from patients suffering from age-related macular degeneration. It is expected that if markers that can be used for diagnosis of age-related macular degeneration and diagnosis methods using such markers can be created, such markers or methods will be widely usable for diagnosis of vascular disorders and, furthermore of ageing itself.

### DISCLOSURE OF THE INVENTION

### PROBLEM TO BE SOLVED BY THE INVENTION

However, the above-mentioned known markers and marker candidates have poor specificity and/or sensitivity and efficient methods for detecting such markers from biological samples have not been established. Because, in general, these markers are not clinically used, there are high demands on markers with higher specificity and sensitivity for ageing and diseases accompanied with vascular disorders. In addition, a high-throughput assay method that is less stressful for patients has been awaited, whereby degrees of pathological progression, as well as post-surgical time-dependent changes, can be objectively/quantitatively determined for patients.

An object of the present invention is to provide a composition or kit useful for diagnosis of ageing and a disease accompanied with a vascular disorder, particularly age-related macular degeneration, and a method for assaying ageing and a disease accompanied with vascular disorder using the composition or kit.

### MEANS FOR SOLVING THE PROBLEM

The present inventors have now found blood protein markers specifically detected in age-related macular degeneration patients by subjecting blood specimens of patients with age-related macular degeneration and blood specimens of patients with another ocular diseases, to proteome analysis. This finding led to the complesion of an invention drawn to a method for diagnosing or detecting ageing and a disease accompanied with a vascular disorder using said protein markers.

### <Summary of the Invention>

The present invention has the following characteristics.

(1) A method for assaying ageing, comprising quantitatively or qualitatively measuring and/or detecting one or more of polypeptides comprising any of amino acid sequences shown in SEQ ID NOS: 1 to 21, mutants thereof, or fragments thereof, in a biological sample from a subject.

(2) The method according to (1), wherein ageing is a vascular disorder.

(3) The method according to (2), wherein the vascular disorder is bleeding or edema accompanied with angiogenesis.

(4) The method according to (3), wherein the vascular disorder is bleeding or edema accompanied with angiogenesis in an ocular tissue.

(5) The method according to (4), wherein the bleeding or edema accompanied with angiogenesis in an ocular tissue is age-related macular degeneration.

(6) The method according to any one of (1) to (5), wherein the measurement and/or detection of the polypeptide, a mutant thereof, or a fragment thereof is carried out by mass spectrometry.

(7) The method according to (6), wherein the measurement and/or detection is carried out using a substance capable of binding to the polypeptide, a mutant thereof, or a fragment thereof.

(8) The method according to (7), wherein the substance capable of binding is an antibody or an antigen-binding fragment thereof.

(9) The method according to (8), wherein the antibody labeled with any of an enzyme, a fluorophor, a dye, a radioisotope, or biotin is used.

(10) The method according to (8) or (9), wherein the antibody or an antigen-binding fragment thereof is a monoclonal antibody, a polyclonal antibody, or an antigen-binding fragment thereof.

(11) The method according to any one of (1) to (10), wherein the biological sample is blood, plasma, or serum.

(12) A composition for diagnosis and/or detection of ageing, which comprises one or more antibody probes selected from antibodies, antigen-binding fragments thereof, or chemically modified derivatives thereof capable of specifically binding to at least one of polypeptides comprising any of amino acid sequences shown in SEQ ID NOS: 1 to 21, mutants thereof, or fragments thereof.

(13) A kit for diagnosis and/or detection of ageing, which comprises one or more antibody probes selected from antibodies, antigen-binding fragments thereof, or chemically modified derivatives thereof capable of specifically binding to at least one of polypeptides comprising any of amino acid sequences shown in SEQ ID NOS: 1 to 21, mutants thereof, or fragments thereof.

(14) The composition or kit according to (12) or (13), wherein ageing is a vascular disorder.

(15) The composition or kit according to (14), wherein the vascular disorder is bleeding or edema accompanied with angiogenesis.

(16) The composition or kit according to (15), wherein the vascular disorder is bleeding or edema accompanied with angiogenesis in an ocular tissue.

(17) The composition or kit according to (16), wherein the bleeding or edema accompanied with angiogenesis in an ocular tissue is age-related macular degeneration.

(18) Use of one or more antibody probes selected from antibodies, antigen-binding fragments thereof, or chemically modified derivatives thereof capable of specifically binding to at least one of polypeptides comprising any of amino acid sequences shown in SEQ ID NOS: 1 to 21, mutants thereof, or fragments thereof, in production of the kit according to any one of (13)-(17).

### <Definition>

Terms as used herein comprise definitions as described below.

Herein, mutants of polypeptides comprising any of amino acid sequences shown in SEQ ID NOS: 1 to 21 correspond to mutants comprising a deletion(s), substitution(s), addition(s), or insertion(s) of one or more, preferably one or several, amino acids in the amino acid sequences shown in SEQ ID NOS: 1-21 or partial sequences thereof; or mutants comprising amino acid sequences showing about 80% or more, about 85% or more, preferably about 90% or more, more preferably about 95% or more, about 97% or more, about 98% or more, or about 99% or more identity with the amino acid sequences or partial sequences thereof.

The term "% identity" as used herein generally refers to the percentage (%) of the number of amino acid residues or positions that are identical in two amino acid seqeunces relative to the total number of amino acid residues or positions in two amino acid sequences represented when the two amino acid sequences are aligned with or without introduction of a gap. The identity between the two amino acid sequences can be determined using a mathamaticl algorithm. Examples of such algorithm include an algorithm as described in Karlin and Altshul, Proc. Natl. Acad. Sci. USA 1990, 87: 2264 and an improved algorithm as described in Karlin and Altshul, Proc. Natl. Acad. Sci. USA 1993, 90: 5873-5877. These types of algorithms are incorporated in BLASTN, BLASTX, and the like (Altshul et al., J. Mol. Biol. 1990, 215:403). In order to obtain an amino acid sequence homologous to any one of the polypeptide amino acid sequences shown in SEQ ID NOS: 1 to 21, a BLAST protein search is carried out using the BLAST program (e.g., score = 50; word length = 3). In addition, gapped BLAST (Altshul et al., Nucleic Acid Res. 1997, 25: 3389) can be used to obtain a gapped alignment.

The term "several" as used herein refers to an integer of 10, 9, 8, 7, 6, 5, 4, 3, or 2.

The term "chemically modified derivative" as used herein refers to, but is not limited to, a derivative labeled with a label such as enzyme, fluorophor, dye, or radioisotope, or a derivative having chemical modification such as biotinylation, acetylation, glycosylation, phosphorylation, ubiquitination, or sulfation.

The term "composition or kit for diagnosis and/or detection" as used herein refers to a composition or kit that can be directly or indirectly used for: diagnosing and/or detecting the presence or absence of affection with ageing and a disease accompanied with a vascular disorder such as age-related macular degeneration, the degree of affection, the presence or absence of improvement, or the degree of improvement; or screening for a candidate substance useful for prevention, improvement, or treatment of ageing and a disease accompanied with vascular disorder such as age-related macular degeneration.

The term "biological sample" used herein as a subject of detection or diagnosis refers to a sample that contains, or suspected of containing, a target polypeptide that appears along with ageing and the development of a disease accompanied with a vascular disorder such as age-related macular degeneration, taken from a living body (e.g., cells, tissue, or body fluid (e.g., blood, lymphatic fluid, or urine)).

The term "specifically binding to" as used herein means that an antibody or an antigen-binding fragment thereof forms an antigen-antibody complex with only a target polypeptide (that is, an age-related macular degeneration marker in the present invention), a mutant thereof, or a fragment thereof, but does not substantially form such complexes with other peptidic or polypeptidic substances. As used herein, the term "substantially" means that non-specific formation of such complexes may take place, but to a minor extent.

### ADVANTAGE OF THE INVENTION

The markers for ageing and a disease accompanied with a vascular disorder such as age-related macular degeneration as defined in the present invention are found in a biological sample such as blood of a patient with age-related macular degeneration, but are almost not or are not found in the same of a patient with a different ocular disease such as cataract or glaucoma. The simple use of the presence or amount of said markers as an indicator provides a significant advantage that a disease accompanied with age-related macular degeneration, ageing, and a disease accompanied with a vascular disorder can be easily detected using blood, for example.

This description includes all or part of the contents as disclosed in the description and/or drawings of Japanese Patent Application No. 2008-092245 to which the present application claims a priority.

### BEST MODES FOR CARRYING OUT THE INVENTION

The present invention will be further described specifically as follows.

### <Markers for ageing and a disease accompanied with a vascular disorder>

According to the present invention, markers for diagnosis and/or detection of ageing and a disease accompanied with (or associated with) a vascular disorder using the composition or kit for diagnosis or detection of ageing and a disease accompanied with a vascular disorder such as age-related macular degeneration are polypeptides comprising any of amino acid sequences shown in SEQ ID NOS: 1 to 21, mutants thereof, or fragments thereof.

The polypeptides comprising the amino acid sequences shown in SEQ ID NOS: 1 to 21 of the present invention are listed in Table 1 below with their protein numbers (Swiss-Prot accession names and numbers.) and their properties. These polypeptides were specifically detected in plasma from patients with age-related macular degeneration, whereas they were not detected in plasmas from patients with cataract or glaucoma or they were detected at significantly lower levels in plasmas from patients with cataract or glaucoma than in plasmas from patients with age-related macular degeneration. In addition, the amino acid sequences of these polypeptides as shown in the attached SEQUENCE LISTING are available by accessing the Swiss-Prot data bank or the like.

**[Table 1]**

| SEQ ID NO: | Gene name | Protein No. | Properties |
|---|---|---|---|
| 1 | KRT6B | P04259 | Keratin, type II cytoskeletal 6B |
| 2 | KRT5 | P13647 | Keratin, type II cytoskeletal 5 |
| 3 | RPS27A, UBA52, UBB, UBC | P62988 | Ubiquitin |
| 4 | KRT14 | P02533 | Keratin, type I cytoskeletal 14 |
| 5 | KRT16 | P08779 | Keratin, type I cytoskeletal 16 |
| 6 | PCLO | Q9Y6V0 | protein piccolo |
| 7 | FN1 | P02751 | Fibronectin |
| 8 | SPP1 | P10451 | Osteopontin |
| 9 | LCN2 | P80188 | Neutrophil gelatinase-associated lipocalin |
| 10 | PDLIM7 | Q9NR12 | PDZ and LIM domain protein 7 |
| 11 | DYNC1I2 | Q13409 | Cytoplasmic dynein 1 intermedite chain 2 |
| 12 | GUCA2A | Q02747 | Guanylin |
| 13 | DSC1 | Q08554 | Desmocollin-1 |
| 14 | NUP210 | Q8TEM1 | Nuclear pore membrane glycoprotein 210 |
| 15 | BPTF | Q12830 | Nucleosome-remodeling factor subunit BPTF |
| 16 | DCD | P81605 | Dermcidin |
| 17 | - | P01623 | Ig kappa chain V-III region WOL |
| 18 | CFL1 | P23528 | Cofilin-1 |
| 19 | - | P06314 | Ig kappa chain V-IV region B17 |
| 20 | HIST1H1E | P10412 | Histone H1.4 |
| 21 | FBXL19 | Q6PCT2 | F-box/LRR-repeat protein 19 |

In the present invention, all of the above target polypeptides for detection of ageing and a disease accompanied with a vascular disorder are characterized in that the polypeptides can be detected only in plasmas of age-related macular degeneration patients, or that the levels of the polypeptides in age-related macular degeneration patients are significantly or remarkably higher than those in cataract or glaucoma patients. As used herein, the term "significantly" refers to the presence of a statistically significant difference, wherein the significance level (p) is less than 0.05.

Therefore, when any one of, preferably two or more of, age-related macular degeneration marker polypeptides is/are detected in a biological sample of a subject, the occurrence of age-related macular degeneration, a disease accompanied with a vascular disorder, and ageing can be determined.

The polypeptides used in the present invention can be prepared by chemical synthesis (e.g., peptide synthesis) or DNA recombination techniques, which are conventionally used in the art. The DNA recombination techniques are preferably used in terms of the ease of procedures or purification.

First, polynucleotide sequences encoding partial sequences of the polypeptides used in the present invention may be chemically synthesized using automated DNA synthesizers. The phosphoramidite method is generally employed for such synthesis, which enables the automatic synthesis of a single-stranded DNA with a length of no more than approximately 100 nucleotides. The automated DNA synthesizers are commercially available from, for example, Polygen or ABI.

With the use of the thus obtained polynucleotides as probes or primers, a cDNA clone of interest is obtained by known cDNA cloning; that is, by constructing a cDNA library via an RT-PCR method from poly A(+)RNA that is obtained by treating total RNA (which is extracted from a tissue of a living body, such as ocular tissue, in which the above target gene is expressed) with an oligo dT cellulose column and then performing screening of the library, such as hybridization screening, expression screening, or antibody screening. If necessary, such cDNA clone can be further amplified by the PCR method. By such procedures, cDNA corresponding to a gene of interest can be obtained.

Probes or primers are selected from sequences of 15 to 100 continuous nucleotides based on the polypeptide sequences shown in SEQ ID NOS: 1-21 and then can be synthesized as described above. Also, cDNA cloning techniques are described in Sambrook, J. and Russel, D., Molecular Cloning, A LABORATORY MANUAL, Cold Spring Harbor Laboratory Press, issued January 15, 2001, Vol. 1, 7.42-7.45 and Vol. 2, 8.9-8.17, and Ausubel et al., Current Protocols in Molecular Biology, 1994, John Wiley & Sons, for example.

Next, the thus obtained cDNA clones are each incorporated into an expression vector, and then prokaryotic or eukaryotic host cells transformed or transfected with the vector are cultured, so that a polypeptide of interest can be obtained from the cells or culture supernatants. In this case, a nucleotide sequence encoding a secretory signal sequence may be flanked at the 5' end of a DNA encoding a mature polypeptide of interest, so that the mature polypeptide can be secreted extracellularly.

Vectors and expression systems are available from Novagen, Takara Shuzo, Daiichi Pure Chemicals, Qiagen, Stratagene, Promega, Roche Diagnositics, Invitrogen, Genetics Institute, and Amersham Bioscience, for example. As host cells, prokaryotic cells such as bacteria (e.g., *Escherichia coli* and *Bacillus subtilis*), yeast (e.g., *Saccharomyces cerevisiae*), insect cells (e.g., Sf cell), mammalian cells (e.g., COS, CHO, BHK, and NIH3T3), and the like can be used. Vectors may contain, in addition to DNA encoding the polypeptide, regulatory elements such as a promoter (e.g., lac promoter, trp promoter, P_{L} promoter, P_{R} promoter, SV40 viral promoter, 3-phosphoglycerate kinase promoter, or glycolytic enzyme promoter), an enhancer, a polyadenylation signal, a ribosomal binding site, a replication origin, a terminator, a selection marker (e.g., a drug resistance gene such as ampicillin resistance gene or tetracycline resistance gene; or a complementary auxotrophic marker such as LEU2 or URA3), and the like.

Also, to facilitate purification of a polypeptide, an expression product can also be generated in the form of a fusion polypeptide wherein a peptidic label is bound to the C-terminus or the N-terminus of the polypeptide. Examples of a typical peptidic label include, but are not limited to, a histidine repeat (His tag) comprising 6 to 10 His residues, FLAG, a myc peptide, and a GFP polypeptide.

When the polypeptides according to the present invention are produced without adding any peptidic label, examples of purification methods include ion exchange chromatography. In addition, a combination of techniques including gel filtration chromatography or hydrophobic chromatography, isoelectric point chromatography, high performance liquid chromatography (HPLC), electrophoresis, ammonium sulfate fractionation, salting-out, ultrafiltration, and dialysis may be used. Furthermore, when a peptidic label such as a histidine repeat, FLAG, myc, or GFP is bound to the polypeptide, the purification is carried out using an affinity chromatography appropriate for each peptidic label that is generally used. In this case, an expression vector that makes isolation and purification easy is preferably constructed. In particular, the expression vector is constructed such that a target polypeptide is expressed in the form of a fusion with peptidic label, and the polypeptide is prepared genetic engineeringly using the vector. By doing so, the isolation and purification of the polypeptide can be easily performed.

Purification of nucreic acids can be carried out by purification methods using agalose gel electrophoresis, DNA-binding resin column, and the like. Alternatively, because there are commercially available automated nucleic acid purification systems and nucleic acid purification kits, etc. Purification of nucleic acids may be carried out using such commercially available tools.

As defined above, mutants of the above polypeptides according to the present invention refer to mutants comprising a deletion(s), substitution(s), addition(s), or insertion(s) of one or more, preferably one or several, amino acids in the amino acid sequences shown in SEQ ID NOS: 1-21 or partial sequences thereof; or mutants comprising amino acid sequences showing about 80% or more, about 85% or more, preferably about 90% or more, more preferably about 95% or more, about 97% or more, about 98% or more, or about 99% or more identity with the amino acid sequences or partial sequences thereof. Examples of such mutants include: homologs from mammalian species different from humans; and naturally occurring mutants such as mutants based on polymorphic mutation among mammals of the same species (e.g., race), splice mutants, and natural mutants.

Also, fragments of the polypeptides of the present invention comprise at least 7, at least 8, at least 10, or at least 15, preferably at least 20, or at least 25, more preferably at least 30, at least 40, at least 50, at least 100, at least 150, or at least 200, or all continuous amino acid residues in the amino acid sequences of the polypeptides, and retain one or more epitopes. Such fragments are capable of immunospecifically binding to antibodies or fragments thereof of the present invention. When the above polypeptides are present in blood, for example, it is assumed that the polypeptides are present as a result of cleavage and fragmentation by an enzyme existing therein such as protease or peptidase.

### <A composition or kit for diagnosis or detection of ageing and a disease accompanied with a vascular disorder such as age-related macular degeneration>

According to the present invention, the following is provided: a composition for diagnosis and/or detection of ageing and a disease accompanied with a vascular disorder such as age-related macular degeneration, which comprises one or more, preferably 3 or more, more preferably 5 or more, further preferably 10 or more, and most preferably 21 different antibody probes selected from among antibodies, antigen-binding fragments, or chemically modified derivatives thereof capable of specifically binding to polypeptides comprising any of amino acid sequences shown in SEQ ID NOS: 1 to 21, mutants thereof, or fragments thereof.

As used herein, the term "composition" refers not only to a simple mixture of a plurality of antibody probes but also to a combination of the same.

An antibody that recognizes a polypeptide which is an age-related macular degeneration marker is capable of specifically binding to the polypeptide via an antigen binding site of the antibody. Such antibody usable in the present invention can be prepared by conventional techniques using polypeptides having the amino acid sequences of SEQ ID NOS: 1-21, mutants thereof, or fragments thereof or using a fusion polypeptide(s) thereof, as one or more immunogens. Examples of these polypeptides, mutants thereof, fragments thereof, or fusion polypeptides include epitopes that induce antibody formation. These epitopes may be linear epitopes or epitopes with higher order structures (discontinuous epitopes). In general, an epitope capable of binding to an antibody is thought to exist on the hydrophilic surface of a polypeptide structure.

Examples of antibodies that can be used in the present invention include antibodies of any types, classes, and subclasses. Examples of such antibodies include IgG, IgE, IgM, IgD, IgA, IgY, IgG1, IgG2, IgG3, IgG4, IgA1, and IgA2.

Moreover, antibodies in all forms are induced by the polypeptides according to the present invention. When the whole or part of the polypeptide or an epitope has been isolated, both polyclonal antibody and monoclonal antibody can be prepared using conventional techniques. An example of such method is as described in Monoclonal Antibodies, Hybridomas: A New Dimension in Biological Analyses, supervised by Kennet et al., Ple num Press, New York, 1980, for example.

A polyclonal antibody can be prepared by immunizing animals such as birds (e.g., chicken) and mammals (e.g., rabbit, goat, horse, sheep, and mouse) with the polypeptide according to the present invention. The antibody of interest can be purified from the blood of immunized animals through an appropriate combination of techniques such as ammonium sulfate fractionation, ion exchange chromatography, and affinity chromatography.

A monoclonal antibody can be obtained by a technique that comprises producing a hybridoma cell line that produces a monoclonal antibody specific to each polypeptide in mice by conventional techniques. One method for producing such hybridoma cell line comprises immunizing animals with the polypeptide according to the present invention, collecting spleen cells from immunized animals, fusing the spleen cells to a myeloma cell line so as to generate hybridoma cells, and then identifying the hybridoma cell line that produces a monoclonal antibody binding to the polypeptide. The monoclonal antibody can be collected by conventional techniques.

Preparation of monoclonal and polyclonal antibodies is described in detail as follows.

### A. Preparation of monoclonal antibody

### (1) Immunization and collection of antibody-producing cell

An immunogen obtained as described above is administered to a mammal such as a rat, a mouse (e.g., the inbred mouse strain Balb/c), or a rabbit. The dose of the immunogen can be appropriately determined depending on, for example, the type of an animal to be immunized or the route of administration, and it is about 50 µg to 200 µg per animal. Immunization is primarily performed by injecting an immunogen subcutaneously or intraperitoneally. Also, the intervals of immunization are not particularly limited. After the primary immunization, boost immunization is carried out 2 to 10 times, and preferably 3 or 4 times, at intervals of several days to several weeks, and preferably at intervals of 1 to 4 weeks. After the primary immunization, the antibody titer of the blood serum of the immunized animal is repeatedly measured by, for example, ELISA (Enzyme-Linked Immuno Sorbent Assay). When the antibody titer reaches a plateau, the immunogen is injected intravenously or intraperitoneally to complete the final immunization. Antibody-producing cells are collected 2 to 5 days and preferably 3 days after the final immunization. Examples of antibody-producing cells include spleen cells, lymph node cells, and peripheral blood cells, and preferably spleen cells or regional lymph node cells.

### (2) Cell fusion

Hybridoma cell lines that produce monoclonal antibodies specific to each protein can be produced and then identified by conventional techniques. A method for producing such hybridoma cell lines comprises immunizing an animal with the polypeptide of the invention, removing spleen cells from the immunized animal, fusing the spleen cells to a myeloma cell line, producing hybridoma cells therefrom, and then identifying a hybridoma cell line that produces a monoclonal antibody binding to the polypeptide of interest. Myeloma cell lines to be fused to antibody-producing cells, which can be used herein, are commercially available established cell lines of animals such as mice. Preferably, cell lines to be used herein have drug selectivity so that they cannot survive in a HAT selective medium (containing hypoxanthine, aminopterin, and thymidine) in an unfused state, but they can survive only in a state fused to antibody-producing cells. Such established cell lines are preferably derived from an animal of the same species with the immunized animal. A specific example of the myeloma cell line is a P3X63-Ag.8 strain (ATCC TIB9), which is a BALB/c mouse-derived hypoxanthine·guanine·phosphoribosyl·transferase (HGPRT) deficient cell line.

Subsequently, the myeloma cell lines are fused to the antibody-producing cells. Cell fusion is carried out in a serum-free medium for animal cell culture, such as DMEM or RPMI-1640 medium, by mixing the antibody-producing cells with the myeloma cell lines at about 1:1 to 20:1 in the presence of a cell fusion accelerator. As the cell fusion accelerator, polyethylene glycol or the like having an average molecular weight ranging from 1,500 to 4,000 daltons can be used at a concentration ranging from about 10% to 80%, for example. Optionally, an auxiliary agent, such as dimethyl sulfoxide, can be used in combination to enhance the fusion efficiency. Further, the antibody-producing cells can be fused to the myeloma cell lines using a commercially available cell fusion apparatus utilizing electric stimuli (e.g., electroporation).

### (3) Selection and cloning of hybridoma

The hybridomas of interest are selected from the fused cells. To this end, the cell suspension is adequately diluted with, for example, a fetal bovine serum-containing RPMI-1640 medium, then the suspension is aliquoted into each well of a microtiter plate at about two million cells/well, a selection medium is added to each well, and then culture is carried out while appropriately exchanging the selection medium with the same fresh medium. The culture temperature ranges from 20°C to 40°C and is preferably about 37°C. When the myeloma cell is an HGPRT-deficient cell line or thymidine kinase-deficient cell line, only a hybridoma of a cell having an ability to produce an antibody and a myeloma cell line can selectively be cultured and grown in the selection medium containing hypoxanthine, aminopterin, and thymidine (i.e., the HAT medium). As a result, cells that start to grow on about day 14 after the initiation of culture in the selection medium can be obtained as hybridoma cells.

Subsequently, whether or not the culture supernatant of the grown hybridomas contains the antibody of interest is screened for. Screening of hybridomas can be carried out in accordance with conventional techniques, without particular limitation. For example, the culture supernatant in a well containing the grown hybridomas is partially sampled and then subjected to enzyme immuno assay (EIA) or ELISA or radio immuno assay (RIA). The fused cells are cloned using the limiting dilution method or the like, and monoclonal antibody-producing cells, i.e. hybridomas, are established in the end. The hybridoma is stable during culture in a basic medium, such as RPMI-1640 or DMEM, and the hybridoma can produce and secrete a monoclonal antibody that reacts specifically with a polypeptidic marker for age-related macular degeneration of the present invention.

### (4) Recovery of antibody

Monoclonal antibodies can be recovered by conventional techniques. Specifically, a monoclonal antibody can be collected from the established hybridoma by a conventional cell culture technique, ascites development, or the like. According to the cell culture technique, hybridomas are cultured in an animal cell culture medium, such as 10% fetal bovine serum-containing RPMI-1640 medium, MEM medium, or a serum-free medium, under common culture conditions (e.g., 37°C, 5% CO₂ concentration) for 2 to 10 days, and the antibody is obtained from the culture supernatant. In the case of ascites development, about 10 millions of hybridoma cells are administered intraperitoneally to an animal of the same species as the mammal from which the myeloma cells are derived, so as to allow the hybridoma cells to grow in large quantity. After one to two weeks, the ascites or blood serum is taken from the animal.

Where antibody purification is required in the above-described method for collecting the antibody, known techniques, such as salting out with ammonium sulfate, ion-exchange chromatography, affinity chromatography, and gel filtration chromatography, may be appropriately selected or combined to obtain the purified monoclonal antibody of the present invention.

### B. Preparation of polyclonal antibody

When polyclonal antibodies are prepared, an animal is immunized in the same manner as described above, the antibody titer is measured on days 6 to 60 after the final immunization by enzyme immuno assay (EIA or ELISA) or radio immuno assay (RIA), and blood is taken on the day when the maximal antibody titer is measured, in order to obtain antiserum. Thereafter, the reactivity of the polyclonal antibodies in the antiserum is measureed by ELISA or the like.

Also, in the present invention, an antigen-binding fragment of the above antibodies can also be used. Examples of antigen-binding fragments that can be produced by conventional techniques include, but are not limited to, Fab and F(ab')₂, Fv, scFv, and dsFv. Examples thereof also include antibody fragments and derivatives thereof that can be produced by genetic engineering techniques. Examples of such antibodies include synthetic antibodies, recombinant antibodies, multi-specific antibodies (including bispecific antibodies), and single chain antibodies.

The antibodies of the present invention can be used *in vitro* and *in vivo.* In the present invention, the antibodies can be used in assays for detection of the presence of polypeptides or (poly)peptide fragments thereof. A monoclonal antibody is preferably used to enable specific detection in the assay. Even in the case of a polyclonal antibody, a specific antibody can be obtained by a so-called absorption method that comprises binding an antibody to an affinity column to which a purified polypeptide is bound.

Therefore, the composition of the present invention can contain at least one, preferably a plural number of types of (e.g., two or three types or more), and more preferably all types of antibodies or antigen-binding fragments thereof capable of specifically binding to the polypeptides comprising the amino acid sequences of SEQ ID NOS: 1-21, mutants thereof, or fragments thereof.

A label, such as a fluorophore, an enzyme, or a radioisotope may be bound to an antibody or an antigen-binding fragment thereof to be used in the present invention, if necessary. Alternatively, such label may be bound to a secondary antibody.

Examples of a fluorophore include fluorescein and a derivative thereof, rhodamine and a derivative thereof, dansyl chloride and a derivative thereof, and umbelliferone.

Examples of an enzyme include horseradish peroxidase and alkaline phosphatase.

Examples of a radioisotope include iodines (¹³¹I, ¹²⁵ I, ¹²³I, and ¹²¹I) phosphorus (³²P), sulfur (³⁵S), and metals (e.g., ⁶⁸Ga, ⁶⁷Ga, ⁶⁸Ge, ⁵⁴Mn, ⁹⁹Mo, ⁹⁹Tc, and ¹³³Xe).

Examples of other labels include luminescence substances such as luminol and bioluminescence substances such as luciferase and luciferin.

Also, if necessary, an avidin-biotin system or a streptavidin-biotin system can also be used herein. In this case, for example, biotin can be bound to the antibody or an antigen-binding fragment thereof of the present invention.

The present invention further provides a kit for diagnosis and/or detection of ageing, which comprises one or more antibody probes selected from antibodies or antigen-binding fragments thereof, or chemically modified derivatives thereof capable of specifically binding to at least one polypeptide comprising any of the amino acid sequences shown in SEQ ID NOS: 1 to 21, a mutant thereof, or a fragment thereof.

In this context, the present invention further provides a use of one or more antibody probes selected from antibodies or antigen-binding fragments thereof, or chemically modified derivatives thereof capable of specifically binding to at least one polypeptide comprising any of the amino acid sequences shown in SEQ ID NOS: 1 to 21, a mutant thereof, or a fragment thereof, in production of the above-described kit.

As used herein, the term "ageing" includes a vascular disorder. The vascular disorder includes bleeding or edema accompanied with angiogenesis, for example, bleeding or edema accompanied with angiogenesis in ocular tissue, such as age-related macular degeneration.

The kit comprises, for example, individual containers (e.g., vials) in which the above-described antibody probes for detection of markers of ageing and a disease accompanied with a vascular disorder are packaged individually or, appropriately, in admixture. Preferably, antibody probes may be packaged in the lyophilized state in containers.

Alternatively, the kit of the present invention may comprise a solid-phase support comprising a multi-well plate, an array, a microtiter plate, a test piece (or test strip), spherical carriers such as latex beads or magnetic beads, or the like, to which antibodies or fragments thereof capable of specifically binding to the aforementioned polypeptides have been attached or (covalently or non-covalently) bonded.

Further, the kit of the present invention may contain a buffer, a secondary antibody, instructions, and the like, which are used in the assay method of the present invention.

Instead of the above-described antibody probes, nucleic acid probes can be used in the method, composition, and kit of the present invention. The nucleic acid probes are DNAs, which code for polypeptides comprising any of amino acid sequences shown in SEQ ID NOS: 1-21 as described above, mutants thereof, or fragments thereof. The nucleic acid probes can be produced by the above-described method for producing the corresponding polypeptides by gene recombination technology. Mutants or fragments thereof can be produced by, for example, PCR using appropriate primers and parent polypeptides as templates. The nucleic acid probes can generally have a size of approximately 15-100 nucleotides or more and preferably approximately 20-80 nucleotides. In addition, with the use of nucleic acid probes, DNA-DNA hybridization, DNA-RNA hybridization, RNA-RNA hybridization, or the like is performed under stringent conditions such that target markers are detected. Regarding hybridization conditions, for example, conditions described in Sambrook, J. and Russel, D., Molecular Cloning, A LABORATORY MANUAL, Cold Spring Harbor Laboratory Press, published on January 15, 2001, Vol. 1, 7.42-7.45, Vol. 2, 8.9-8.17, or Ausubel et al., Current Protocols in Molecular Biology, 1994, John Wiley & Sons, etc. can be employed.

### <Detection of ageing and a disease accompanied with a vascular disorder>

According to the present invention, ageing, and a disease accompanied with vascular disorder, can be detected by a method that comprises determining *in vitro* the presence or amount of one or more of the polypeptides shown in SEQ ID NOS: 1-21, mutants thereof, or fragments thereof in a biological sample from a subject using substances capable of binding to the above-described markers. In a possible diagnosis conducted by the method of the present invention, where the age-related macular degeneration marker(s) is/are detected or the gene expression levels are determined to be significantly higher than control levels, a subject is determined to be in the advanced stage of ageing or a vascular disorder, thus to suffer from age-related macular degeneration.

In the method of the present invention, the detection of markers for ageing and a disease accompanied with vascular disorder may be performed using a single marker, but is preferably performed using a plurality of (e.g., from 2 or more, 3 or more, 4 or more, or 5 or more, to 21) markers. This is intended to avoid unpredictable detection of a non-specific complex, in other words, misdiagnosis.

The composition or kit of the present invention is useful for diagnosis, determination, or detection of ageing and a disease accompanied with a vascular disorder, i.e., for diagnosis of the presence or absence of the disease or the degree of the disease. In diagnosis of ageing and a disease accompanied with a vascular disorder, comparison is made with negative controls such as normal cells, normal tissues, or normal body fluids, and then the presence or amount of the above-described age-related macular degeneration markers in a biological sample from a subject is detected. When a difference in the presence or amount is found to be significant, the subject is suspected of ageing, advanced vascular disorder, or suffering from age-related macular degeneration.

Examples of test samples used in the present invention include body fluids such as blood, serum, blood plasma, and urine.

Examples of the above-described substances capable of binding to age-related macular degeneration markers include not only the above-described antibodies or antigen-binding fragments thereof but also, for example, aptamers, Affibody^{™} (Affibody), receptors of the age-related macular degeneration markers, substances inhibiting the specific action of the age-related macular degeneration markers, and substances activating the specific action of the age-related macular degeneration markers, preferably antibodies or antigen-binding fragments thereof or chemically modified derivatives thereof.

In an embodiment of the present invention, the measurement can comprise the steps of: bringing an antibody or fragment thereof, which may be optionally labeled with a conventional enzyme or fluorophore, into contact with a tissue section or a homogenized tissue or a body fluid; and qualitatively or quantitatively measuring an antigen-antibody complex. The detection is carried out by, for example, a method for measuring the presence and the level of a target polypeptide by immunoelectron microscopy, or a method for measuring the presence or the levels of target polypeptides by a conventional method, such as an enzyme antibody method (e.g., ELISA), a fluorescent antibody technique, a radioimmunoassay, a homogeneous method, a heterogeneous method, a solid phase method, or a sandwich method. Where the target polypeptide is found to be present in a body fluid or an advanced vascular disorder tissue or cells, preferably blood, obtained from a subject, or the level of the target polypeptide is found to be significantly increased or higher than the negative control level, the subject is determined to have ageing and a disease accompanied with vascular disorder. As used herein, the term "significantly" refers to the presence of a statistically significant difference (p<0.05).

An example of a measurement method alternative for an immunological method is a method using mass spectrometry. This method can be performed by procedures described in Examples later. Specifically, a biological sample, such as serum or blood plasma, is filtered using a filter to remove contaminants, diluted with a buffer (e.g., pH, about 8), and then adjusted to have a concentration ranging from about 10 mg/ml to about 15 mg/ml. Subsequently, the resultant is filtered through a hollow fiber filter (Reference Example (1) below) or a centrifugal flat membrane filter, which is capable of removing proteins with a molecular weight of 50,000 or more, so as to perform molecular weight fractionation. The fractions are treated with protease (e.g., trypsin) for peptidization and then the resultants are subjected to a mass spectrometer (the type using matrix-assisted laser desorption ionization or electrospray ionization). Differences between the amount of a polypeptide existing in a sample of a patient with age-related macular degeneration and the same of a healthy subject or a patient with a different ocular disease can be measured based on the mass-to-charge ratio (m/z) and intensity at a specific peak from the polypeptide of interest.

### EXAMPLES

The present invention will be described in more detail with reference to the examples set forth below; however, the present invention is not limited to the examples.

### <Reference Example>

### (1) Preparation of hollow fiber filter

A hundred polysulfone hollow fibers having a pore size (molecular weight cut off) of approximately 50,000 on the membrane surface were packed into a bundle. The both ends of the bundle were fixed to a glass tube using an epoxy-based potting agent so as not to occlude the hollow parts of the hollow fibers, so that a mini module is prepared. The mini module (module A) was used for removal of high-molecular-weight proteins in serum or blood plasma, having a diameter of about 7 mm and a length of about 17 cm. Similarly, a mini module (module B) to be used for concentrating low-molecular-weight proteins was prepared using a membrane with a pore size (molecular weight cut off) of approximately 3,000. The mini modules have an inlet that is connected to hollow fiber lumen on one end and an outlet on the other end. The inlets and outlets of hollow fibers constitute flow passages of a closed circulatory system formed via a silicon tube. Through the flow passages, a liquid is driven by a Peristar pump to circulate. Also, a glass tube of the hollow fiber mantle is provided with a port for discharging a liquid leaking from the hollow fibers, so that one module set is constituted. The modules were connected to a position in the middle of such flow passage via a "T"-shaped connector, i.e., three modules A and one module B were connected in tandem, thereby forming one hollow fiber filter. The hollow fiber filter was washed with distilled water, and then filled with an aqueous 25 mM ammonium bicarbonate solution (pH 8.2). A fraction raw material (i.e., serum or blood plasma) was injected from the flow passage inlet of the hollow fiber filter and then discharged from the flow passage outlet after fractionation and concentration. Serum or blood plasma injected into the hollow fiber filter was applied to a molecular sieve with a molecular weight cut off of approximately 50,000 for every module A. Thus, components with molecular weights lower than that of 50,000 are concentrated using the module B and then prepared.

### <Example 1>

### (1) Idendification of plasma proteins of age-related macular degeneration patients, cataract patients, and normal tension-glaucoma patients

Heparinized plasmas were obtained for measurement, from 10 patients with age-related macular degeneration (aged 82 on average), 10 patients with cataract and 10 patients with normal tension and glaucoma of similar age. The blood plasmas were centrifuged to remove contaminants, and the resulting plasmas were further diluted with 25 mM ammonium bicarbonate solution (pH 8.2) to a concentration of 12.5 mg/ml, followed by carying out a molecular weight fractionation using the hollow fiber filter as described in Reference Example (1). Each fractionated blood plasma sample (total amount 1.8 ml, comprising 250 µg (max) proteins) was separated into 3 fractions by reversed-phase chromatography with AKTA explorer 10s (GE Healthcare Biosciences). The fractions were each lyophilized and then redissolved in 8 M urea solution. The samples were treated with DTT-iodoacetamide and then diluted 10-fold, followed by overnight digestion at 37°C with trypsin (at a ratio 1:50 of trypsin to proteins) for peptidization. After removal of urea using a desalting column, peptides in each fraction were further fractionated into 8 fractions using ion-exchange column. Each fraction was further fractionated using reverse-phase column, and the eluted peptides were subjected to mass spectrometry with an online-connected mass spectrometer (LCQ Deca XP plus; Thermo Fisher Scientific K.K.).

### (2) Comparison of expressed plasma proteins among age-related macular degeneration patients, cataract patients, and normal tension-glaucoma patients

Data determined in (1) above were analyzed using the protein identification softwares Bioworks (Thermo Fisher Scientific K.K.) and Phenyx (GENE BIO) for comprehensive protein identification. From among identified proteins, proteins identified by the two different types of software were listed and designated as proteins detected from plasma samples of patients with each disease. This was carried out to exclude false-positive proteins contained in analysis results of either one of the softwares by combining the two different softwares having different algorithms. However, unlike Bioworks, Phenyx carries out searching in consideration of isoform-specific amino acid sequences obtained by alternative splicing of an identidal protein and changes in mass after post-translation modification. Hence, the software Phenyx might identify peptides that cannot be identified by Bioworks. Under the above conditions, proteins identified only by Phenyx were also listed.

Among proteins listed for each disease, proteins detected from age-related macular degeneration patients but never from cataract patients or normal tension-glaucoma patients were found as plasma marker proteins. These proteins correspond to polypeptides comprising any of amino acid sequences shown in SEQ ID NOS: 1 to 21 in Table 1 (above) and Sequence Listing. Accordingly, it was revealed that the proteins are useful as age-related macular degeneration markers for detection of age-related macular degeneration or for diagnostic determination of the progression of the same disease during treatment.

### INDUSTRIAL APPLICABILITY

The present invention provides the composition or kit with good specificity and sensitivity for diagnosis of ageing and a disease accompanied with a vascular disorder such as age-related macular degeneration, and it is particularly useful in the pharmaceutical and medical industries.

All publications, patents, and patent applications cited herein are incorporated herein by reference in their entirety.

## Claims

1. A method for assaying ageing, comprising quantitatively or qualitatively measuring and/or detecting one or more of polypeptides comprising any of amino acid sequences shown in SEQ ID NOS: 1 to 21, mutants thereof, or fragments thereof, in a biological sample from a subj ect.

2. The method according to claim 1, wherein ageing is a vascular disorder.

3. The method according to claim 2, wherein the vascular disorder is bleeding or edema accompanied with angiogenesis.

4. The method according to claim 3, wherein the blood disorder is bleeding or edema accompanied with angiogenesis in an ocular tissue.

5. The method according to claim 4, wherein the bleeding or edema accompanied with angiogenesis in an ocular tissue is age-related macular degeneration.

6. The method according to any one of claims 1 to 5, wherein the measurement and/or detection of the polypeptide, a mutant thereof, or a fragment thereof is carried out by mass spectrometry.

7. The method according to claim 6, wherein the measurement and/or detection is carried out using a substance capable of binding to the polypeptide, a mutant thereof, or a fragment thereof.

8. The method according to claim 7, wherein the substance capable of binding is an antibody or an antigen-binding fragment thereof.

9. The method according to claim 8, wherein the antibody labeled with any of an enzyme, a fluorophor, a dye, a radioisotope, or biotin is used.

10. The method according to claim 8 or 9, wherein the antibody or an antigen-binding fragment thereof is a monoclonal antibody, a polyclonal antibody, or an antigen-binding fragment thereof.

11. The method according to any one of claims 1 to 10, wherein the biological sample is blood, plasma, or serum.

12. A composition for diagnosis and/or detection of ageing, which comprises one or more antibody probes selected from antibodies, antigen-binding fragments thereof, or chemically modified derivatives thereof capable of specifically binding to at least one of polypeptides comprising any of amino acid sequences shown in SEQ ID NOS: 1 to 21, mutants thereof, or fragments thereof.

13. A kit for diagnosis and/or detection of ageing, which comprises one or more antibody probes selected from antibodies, antigen-binding fragments thereof, or chemically modified derivatives thereof capable of specifically binding to at least one of polypeptides comprising any of amino acid sequences shown in SEQ ID NOS: 1 to 21, mutants thereof, or fragments thereof.

14. The composition or kit according to claim 12 or 13, wherein the ageing is a vascular disorder.

15. The composition or kit according to claim 14, wherein the vascular disorder is bleeding or edema accompanied with angiogenesis.

16. The composition or kit according to claim 15, wherein the blood disorder is bleeding or edema accompanied with angiogenesis in an ocular tissue.

17. The composition or kit according to claim 16, wherein the bleeding or edema accompanied with angiogenesis in an ocular tissue is age-related macular degeneration.

18. Use of one or more antibody probes selected from antibodies, antigen-binding fragments thereof, or chemically modified derivatives thereof capable of specifically binding to at least one of polypeptides comprising any of amino acid sequences shown in SEQ ID NOS: 1 to 21, mutants thereof, or fragments thereof, in production of the kit according to any one of claims 13-17.


SEQUENCE LISTING

<110>  National Hospital Organization
       Santen Pharmaceutical Co. Ltd.

<120>  Composition, kit, and method for assaying ageing and disease
       accompanied with vascular disorder

<130>  NRS/FP6727226

<140>  09728954.0
<141>  2009-03-31

<150>  PCT/JP2009/056728
<151>  2009-03-31

<150>  JP 2008-092245
<151>  2008-03-31

<160>  21

<170>  PatentIn version 3.4

<210>  1
<211>  564
<212>  PRT
<213>  Homo sapiens

<400>  1

Met Ala Ser Thr Ser Thr Thr Ile Arg Ser His Ser Ser Ser Arg Arg
1               5                   10                  15

Gly Phe Ser Ala Ser Ser Ala Arg Leu Pro Gly Val Ser Arg Ser Gly
            20                  25                  30

Phe Ser Ser Ile Ser Val Ser Arg Ser Arg Gly Ser Gly Gly Leu Gly
        35                  40                  45

Gly Ala Cys Gly Gly Ala Gly Phe Gly Ser Arg Ser Leu Tyr Gly Leu
    50                  55                  60

Gly Gly Ser Lys Arg Ile Ser Ile Gly Gly Gly Ser Cys Ala Ile Ser
65                  70                  75                  80

Gly Gly Tyr Gly Ser Arg Ala Gly Gly Ser Tyr Gly Phe Gly Gly Ala
                85                  90                  95

Gly Ser Gly Phe Gly Phe Gly Gly Gly Ala Gly Ile Gly Phe Gly Leu
            100                 105                 110

Gly Gly Gly Ala Gly Leu Ala Gly Gly Phe Gly Gly Pro Gly Phe Pro
        115                 120                 125

Val Cys Pro Pro Gly Gly Ile Gln Glu Val Thr Val Asn Gln Ser Leu
    130                 135                 140

Leu Thr Pro Leu Asn Leu Gln Ile Asp Pro Ala Ile Gln Arg Val Arg
145                 150                 155                 160

Ala Glu Glu Arg Glu Gln Ile Lys Thr Leu Asn Asn Lys Phe Ala Ser
                165                 170                 175

Phe Ile Asp Lys Val Arg Phe Leu Glu Gln Gln Asn Lys Val Leu Asp
            180                 185                 190

Thr Lys Trp Thr Leu Leu Gln Glu Gln Gly Thr Lys Thr Val Arg Gln
        195                 200                 205

Asn Leu Glu Pro Leu Phe Glu Gln Tyr Ile Asn Asn Leu Arg Arg Gln
    210                 215                 220

Leu Asp Ser Ile Val Gly Glu Arg Gly Arg Leu Asp Ser Glu Leu Arg
225                 230                 235                 240

Asn Met Gln Asp Leu Val Glu Asp Leu Lys Asn Lys Tyr Glu Asp Glu
                245                 250                 255

Ile Asn Lys Arg Thr Ala Ala Glu Asn Glu Phe Val Thr Leu Lys Lys
            260                 265                 270

Asp Val Asp Ala Ala Tyr Met Asn Lys Val Glu Leu Gln Ala Lys Ala
        275                 280                 285

Asp Thr Leu Thr Asp Glu Ile Asn Phe Leu Arg Ala Leu Tyr Asp Ala
    290                 295                 300

Glu Leu Ser Gln Met Gln Thr His Ile Ser Asp Thr Ser Val Val Leu
305                 310                 315                 320

Ser Met Asp Asn Asn Arg Asn Leu Asp Leu Asp Ser Ile Ile Ala Glu
                325                 330                 335

Val Lys Ala Gln Tyr Glu Glu Ile Ala Gln Arg Ser Arg Ala Glu Ala
            340                 345                 350

Glu Ser Trp Tyr Gln Thr Lys Tyr Glu Glu Leu Gln Val Thr Ala Gly
        355                 360                 365

Arg His Gly Asp Asp Leu Arg Asn Thr Lys Gln Glu Ile Ala Glu Ile
    370                 375                 380

Asn Arg Met Ile Gln Arg Leu Arg Ser Glu Ile Asp His Val Lys Lys
385                 390                 395                 400

Gln Cys Ala Asn Leu Gln Ala Ala Ile Ala Asp Ala Glu Gln Arg Gly
                405                 410                 415

Glu Met Ala Leu Lys Asp Ala Lys Asn Lys Leu Glu Gly Leu Glu Asp
            420                 425                 430

Ala Leu Gln Lys Ala Lys Gln Asp Leu Ala Arg Leu Leu Lys Glu Tyr
            435                 440                 445

Gln Glu Leu Met Asn Val Lys Leu Ala Leu Asp Val Glu Ile Ala Thr
    450                 455                 460

Tyr Arg Lys Leu Leu Glu Gly Glu Glu Cys Arg Leu Asn Gly Glu Gly
465                 470                 475                 480

Val Gly Gln Val Asn Ile Ser Val Val Gln Ser Thr Val Ser Ser Gly
                485                 490                 495

Tyr Gly Gly Ala Ser Gly Val Gly Ser Gly Leu Gly Leu Gly Gly Gly
            500                 505                 510

Ser Ser Tyr Ser Tyr Gly Ser Gly Leu Gly Val Gly Gly Gly Phe Ser
            515                 520                 525

Ser Ser Ser Gly Arg Ala Thr Gly Gly Gly Leu Ser Ser Val Gly Gly
    530                 535                 540

Gly Ser Ser Thr Ile Lys Tyr Thr Thr Thr Ser Ser Ser Ser Arg Lys
545                 550                 555                 560

Ser Tyr Lys His



<210>  2
<211>  590
<212>  PRT
<213>  Homo sapiens

<400>  2


<210>  3
<211>  76
<212>  PRT
<213>  Homo sapiens

<400>  3

<210>  4
<211>  472
<212>  PRT
<213>  Homo sapiens

<400>  4

Met Thr Thr Cys Ser Arg Gln Phe Thr Ser Ser Ser Ser Met Lys Gly
1               5                   10                  15

Ser Cys Gly Ile Gly Gly Gly Ile Gly Gly Gly Ser Ser Arg Ile Ser
            20                  25                  30

Ser Val Leu Ala Gly Gly Ser Cys Arg Ala Pro Ser Thr Tyr Gly Gly
        35                  40                  45

Gly Leu Ser Val Ser Ser Ser Arg Phe Ser Ser Gly Gly Ala Tyr Gly
    50                  55                  60

Leu Gly Gly Gly Tyr Gly Gly Gly Phe Ser Ser Ser Ser Ser Ser Phe
65                  70                  75                  80

Gly Ser Gly Phe Gly Gly Gly Tyr Gly Gly Gly Leu Gly Ala Gly Leu
                85                  90                  95

Gly Gly Gly Phe Gly Gly Gly Phe Ala Gly Gly Asp Gly Leu Leu Val
            100                 105                 110

Gly Ser Glu Lys Val Thr Met Gln Asn Leu Asn Asp Arg Leu Ala Ser
        115                 120                 125

Tyr Leu Asp Lys Val Arg Ala Leu Glu Glu Ala Asn Ala Asp Leu Glu
    130                 135                 140

Val Lys Ile Arg Asp Trp Tyr Gln Arg Gln Arg Pro Ala Glu Ile Lys
145                 150                 155                 160

Asp Tyr Ser Pro Tyr Phe Lys Thr Ile Glu Asp Leu Arg Asn Lys Ile
                165                 170                 175

Leu Thr Ala Thr Val Asp Asn Ala Asn Val Leu Leu Gln Ile Asp Asn

            180                 185                 190

Ala Arg Leu Ala Ala Asp Asp Phe Arg Thr Lys Tyr Glu Thr Glu Leu
        195                 200                 205

Asn Leu Arg Met Ser Val Glu Ala Asp Ile Asn Gly Leu Arg Arg Val
    210                 215                 220

Leu Asp Glu Leu Thr Leu Ala Arg Ala Asp Leu Glu Met Gln Ile Glu
225                 230                 235                 240

Ser Leu Lys Glu Glu Leu Ala Tyr Leu Lys Lys Asn His Glu Glu Glu
                245                 250                 255

Met Asn Ala Leu Arg Gly Gln Val Gly Gly Asp Val Asn Val Glu Met
            260                 265                 270

Asp Ala Ala Pro Gly Val Asp Leu Ser Arg Ile Leu Asn Glu Met Arg
        275                 280                 285

Asp Gln Tyr Glu Lys Met Ala Glu Lys Asn Arg Lys Asp Ala Glu Glu
    290                 295                 300

Trp Phe Phe Thr Lys Thr Glu Glu Leu Asn Arg Glu Val Ala Thr Asn
305                 310                 315                 320

Ser Glu Leu Val Gln Ser Gly Lys Ser Glu Ile Ser Glu Leu Arg Arg
                325                 330                 335

Thr Met Gln Asn Leu Glu Ile Glu Leu Gln Ser Gln Leu Ser Met Lys
            340                 345                 350

Ala Ser Leu Glu Asn Ser Leu Glu Glu Thr Lys Gly Arg Tyr Cys Met
        355                 360                 365

Gln Leu Ala Gln Ile Gln Glu Met Ile Gly Ser Val Glu Glu Gln Leu
    370                 375                 380

Ala Gln Leu Arg Cys Glu Met Glu Gln Gln Asn Gln Glu Tyr Lys Ile
385                 390                 395                 400

Leu Leu Asp Val Lys Thr Arg Leu Glu Gln Glu Ile Ala Thr Tyr Arg
                405                 410                 415

Arg Leu Leu Glu Gly Glu Asp Ala His Leu Ser Ser Ser Gln Phe Ser
            420                 425                 430

Ser Gly Ser Gln Ser Ser Arg Asp Val Thr Ser Ser Ser Arg Gln Ile
        435                 440                 445

Arg Thr Lys Val Met Asp Val His Asp Gly Lys Val Val Ser Thr His

450                 455                 460

Glu Gln Val Leu Arg Thr Lys Asn
465                 470

<210>  5
<211>  473
<212>  PRT
<213>  Homo sapiens

<400>  5

<210>  6
<211>  5183

<212>  PRT
<213>  Homo sapiens

<400>  6

Met Gly Asn Glu Ala Ser Leu Glu Gly Glu Gly Leu Pro Glu Gly Leu
1               5                   10                  15

Ala Ala Ala Ala Ala Ala Gly Gly Gly Ala Ser Gly Ala Gly Ser Pro
            20                  25                  30

Ser His Thr Ala Ile Pro Ala Gly Met Glu Ala Asp Leu Ser Gln Leu
        35                  40                  45

Ser Glu Glu Glu Arg Arg Gln Ile Ala Ala Val Met Ser Arg Ala Gln
    50                  55                  60

Gly Leu Pro Lys Gly Ser Val Pro Pro Ala Ala Ala Glu Ser Pro Ser
65                  70                  75                  80

Met His Arg Lys Gln Glu Leu Asp Ser Ser His Pro Pro Lys Gln Ser
                85                  90                  95

Gly Arg Pro Pro Asp Pro Gly Arg Pro Ala Gln Pro Gly Leu Ser Lys
            100                 105                 110

Ser Arg Thr Thr Asp Thr Phe Arg Ser Glu Gln Lys Leu Pro Gly Arg
        115                 120                 125

Ser Pro Ser Thr Ile Ser Leu Lys Glu Ser Lys Ser Arg Thr Asp Leu
    130                 135                 140

Lys Glu Glu His Lys Ser Ser Met Met Pro Gly Phe Leu Ser Glu Val
145                 150                 155                 160

Asn Ala Leu Ser Ala Val Ser Ser Val Val Asn Lys Phe Asn Pro Phe
                165                 170                 175

Asp Leu Ile Ser Asp Ser Glu Ala Ser Gln Glu Glu Thr Thr Lys Lys
            180                 185                 190

Gln Lys Val Val Gln Lys Glu Gln Gly Lys Pro Glu Gly Ile Ile Lys
        195                 200                 205

Pro Pro Leu Gln Gln Gln Pro Pro Lys Pro Ile Pro Lys Gln Gln Gly
    210                 215                 220

Pro Gly Arg Asp Pro Leu Gln Gln Asp Gly Thr Pro Lys Ser Ile Ser
225                 230                 235                 240

Ser Gln Gln Pro Glu Lys Ile Lys Ser Gln Pro Pro Gly Thr Gly Lys
                245                 250                 255

Pro Ile Gln Gly Pro Thr Gln Thr Pro Gln Thr Asp His Ala Lys Leu
            260                 265                 270

Pro Leu Gln Arg Asp Ala Ser Arg Pro Gln Thr Lys Gln Ala Asp Ile
        275                 280                 285

Val Arg Gly Glu Ser Val Lys Pro Ser Leu Pro Ser Pro Ser Lys Pro
    290                 295                 300

Pro Ile Gln Gln Pro Thr Pro Gly Lys Pro Pro Ala Gln Gln Pro Gly
305                 310                 315                 320

His Glu Lys Ser Gln Pro Gly Pro Ala Lys Pro Pro Ala Gln Pro Ser
                325                 330                 335

Gly Leu Thr Lys Pro Leu Ala Gln Gln Pro Gly Thr Val Lys Pro Pro
            340                 345                 350

Val Gln Pro Pro Gly Thr Thr Lys Pro Pro Ala Gln Pro Leu Gly Pro
        355                 360                 365

Ala Lys Pro Pro Ala Gln Gln Thr Gly Ser Glu Lys Pro Ser Ser Glu
    370                 375                 380

Gln Pro Gly Pro Lys Ala Leu Ala Gln Pro Pro Gly Val Gly Lys Thr
385                 390                 395                 400

Pro Ala Gln Gln Pro Gly Pro Ala Lys Pro Pro Thr Gln Gln Val Gly
                405                 410                 415

Thr Pro Lys Pro Leu Ala Gln Gln Pro Gly Leu Gln Ser Pro Ala Lys
            420                 425                 430

Ala Pro Gly Pro Thr Lys Thr Pro Ala Gln Thr Lys Pro Pro Ser Gln
        435                 440                 445

Gln Pro Gly Ser Thr Lys Pro Pro Pro Gln Gln Pro Gly Pro Ala Lys
    450                 455                 460

Pro Ser Pro Gln Gln Pro Gly Ser Thr Lys Pro Pro Ser Gln Gln Pro
465                 470                 475                 480

Gly Ser Ala Lys Pro Ser Ala Gln Gln Pro Ser Pro Ala Lys Pro Ser
                485                 490                 495

Ala Gln Gln Phe Thr Lys Pro Val Ser Gln Thr Gly Phe Gly Lys Pro
            500                 505                 510

Leu Gln Pro Pro Thr Val Ser Pro Ser Ala Lys Gln Pro Pro Ser Gln
        515                 520                 525

Gly Leu Pro Lys Thr Ile Cys Pro Leu Cys Asn Thr Thr Glu Leu Leu
    530                 535                 540

Leu His Val Pro Glu Lys Ala Asn Phe Asn Thr Cys Thr Glu Cys Gln
545                 550                 555                 560

Thr Thr Val Cys Ser Leu Cys Gly Phe Asn Pro Asn Pro His Leu Thr
                565                 570                 575

Glu Ala Lys Glu Trp Leu Cys Leu Asn Cys Gln Met Lys Arg Ala Leu
            580                 585                 590

Gly Gly Asp Leu Ala Pro Val Pro Ser Ser Pro Gln Pro Lys Leu Lys
        595                 600                 605

Thr Ala Pro Val Thr Thr Thr Ser Ala Val Ser Lys Ser Ser Pro Gln
    610                 615                 620

Pro Gln Gln Thr Ser Pro Lys Lys Asp Ala Ala Pro Lys Gln Asp Leu
625                 630                 635                 640

Ser Lys Ala Pro Glu Pro Lys Lys Pro Pro Pro Leu Val Lys Gln Pro
                645                 650                 655

Thr Leu His Gly Ser Pro Ser Ala Lys Ala Lys Gln Pro Pro Glu Ala
            660                 665                 670

Asp Ser Leu Ser Lys Pro Ala Pro Pro Lys Glu Pro Ser Val Pro Ser
        675                 680                 685

Glu Gln Asp Lys Ala Pro Val Ala Asp Asp Lys Pro Lys Gln Pro Lys
    690                 695                 700

Met Val Lys Pro Thr Thr Asp Leu Val Ser Ser Ser Ser Ala Thr Thr
705                 710                 715                 720

Lys Pro Asp Ile Pro Ser Ser Lys Val Gln Ser Gln Ala Glu Glu Lys
                725                 730                 735

Thr Thr Pro Pro Leu Lys Thr Asp Ser Ala Lys Pro Ser Gln Ser Phe
            740                 745                 750

Pro Pro Thr Gly Glu Lys Val Thr Pro Phe Asp Ser Lys Ala Ile Pro
        755                 760                 765

Arg Pro Ala Ser Asp Ser Lys Ile Ile Ser His Pro Gly Pro Ser Ser
    770                 775                 780

Glu Ser Lys Gly Gln Lys Gln Val Asp Pro Val Gln Lys Lys Glu Glu
785                 790                 795                 800

Pro Lys Lys Ala Gln Thr Lys Met Ser Pro Lys Pro Asp Ala Lys Pro
                805                 810                 815

Met Pro Lys Gly Ser Pro Thr Pro Pro Gly Pro Arg Pro Thr Ala Gly
            820                 825                 830

Gln Thr Val Pro Thr Pro Gln Gln Ser Pro Lys Pro Gln Glu Gln Ser
        835                 840                 845

Arg Arg Phe Ser Leu Asn Leu Gly Ser Ile Thr Asp Ala Pro Lys Ser
    850                 855                 860

Gln Pro Thr Thr Pro Gln Glu Thr Val Thr Gly Lys Leu Phe Gly Phe
865                 870                 875                 880

Gly Ala Ser Ile Phe Ser Gln Ala Ser Asn Leu Ile Ser Thr Ala Gly
                885                 890                 895

Gln Pro Gly Pro His Ser Gln Ser Gly Pro Gly Ala Pro Met Lys Gln
            900                 905                 910

Ala Pro Ala Pro Ser Gln Pro Pro Thr Ser Gln Gly Pro Pro Lys Ser
        915                 920                 925

Thr Gly Gln Ala Pro Pro Ala Pro Ala Lys Ser Ile Pro Val Lys Lys
    930                 935                 940

Glu Thr Lys Ala Pro Ala Ala Glu Lys Leu Glu Pro Lys Ala Glu Gln
945                 950                 955                 960

Ala Pro Thr Val Lys Arg Thr Glu Thr Glu Lys Lys Pro Pro Pro Ile
                965                 970                 975

Lys Asp Ser Lys Ser Leu Thr Ala Glu Pro Gln Lys Ala Val Leu Pro
            980                 985                 990

Thr Lys Leu Glu Lys Ser Pro Lys  Pro Glu Ser Thr Cys  Pro Leu Cys
        995                 1000                1005

Lys Thr  Glu Leu Asn Ile Gly  Ser Lys Asp Pro Pro  Asn Phe Asn
    1010                1015                1020

Thr Cys  Thr Glu Cys Lys Asn  Gln Val Cys Asn Leu  Cys Gly Phe
    1025                1030                1035

Asn Pro  Thr Pro His Leu Thr  Glu Asn Cys Gln Thr  Gln Arg Ala
    1040                1045                1050

Ile Ser  Gly Gln Leu Gly Asp  Ile Arg Lys Met Pro  Pro Ala Pro
    1055                1060                1065

Ser Gly Pro Lys Ala Ser Pro Met Pro Val Pro Thr Glu Ser Ser
    1070                1075                1080

Ser Gln Lys Thr Ala Val Pro Pro Gln Val Lys Leu Val Lys Lys
    1085                1090                1095

Gln Glu Gln Glu Val Lys Thr Glu Ala Glu Lys Val Ile Leu Glu
    1100                1105                1110

Lys Val Lys Glu Thr Leu Ser Met Glu Lys Ile Pro Pro Met Val
    1115                1120                1125

Thr Thr Asp Gln Lys Gln Glu Glu Ser Lys Leu Glu Lys Asp Lys
    1130                1135                1140

Ala Ser Ala Leu Gln Glu Lys Lys Pro Leu Pro Glu Glu Lys Lys
    1145                1150                1155

Leu Ile Pro Glu Glu Glu Lys Ile Arg Ser Glu Glu Lys Lys Pro
    1160                1165                1170

Leu Leu Glu Glu Lys Lys Pro Thr Pro Glu Asp Lys Lys Leu Leu
    1175                1180                1185

Pro Glu Ala Lys Thr Ser Ala Pro Glu Glu Gln Lys His Asp Leu
    1190                1195                1200

Leu Lys Ser Gln Val Gln Ile Ala Glu Glu Lys Leu Glu Gly Arg
    1205                1210                1215

Val Ala Pro Lys Thr Val Gln Glu Gly Lys Gln Pro Gln Thr Lys
    1220                1225                1230

Met Glu Gly Leu Pro Ser Gly Thr Pro Gln Ser Leu Pro Lys Glu
    1235                1240                1245

Asp Asp Lys Thr Thr Lys Thr Ile Lys Glu Gln Pro Gln Pro Pro
    1250                1255                1260

Cys Thr Ala Lys Pro Asp Gln Glu Lys Glu Asp Asp Lys Ser Asp
    1265                1270                1275

Thr Ser Ser Ser Gln Gln Pro Lys Ser Pro Gln Gly Leu Ser Asp
    1280                1285                1290

Thr Gly Tyr Ser Ser Asp Gly Ile Ser Ser Ser Leu Gly Glu Ile
    1295                1300                1305

Pro Ser Leu Ile Pro Thr Asp Glu Lys Asp Ile Leu Lys Gly Leu
    1310                1315                1320

Lys Lys Asp Ser Phe Ser Gln Glu Ser Ser Pro Ser Ser Pro Ser
    1325                1330                1335

Asp Leu Ala Lys Leu Glu Ser Thr Val Leu Ser Ile Leu Glu Ala
    1340                1345                1350

Gln Ala Ser Thr Leu Ala Asp Glu Lys Ser Glu Lys Lys Thr Gln
    1355                1360                1365

Pro His Glu Val Ser Pro Glu Gln Pro Lys Asp Gln Glu Lys Thr
    1370                1375                1380

Gln Ser Leu Ser Glu Thr Leu Glu Ile Thr Ile Ser Glu Glu Glu
    1385                1390                1395

Ile Lys Glu Ser Gln Glu Glu Arg Lys Asp Thr Phe Lys Lys Asp
    1400                1405                1410

Ser Gln Gln Asp Ile Pro Ser Ser Lys Asp His Lys Glu Lys Ser
    1415                1420                1425

Glu Phe Val Asp Asp Ile Thr Thr Arg Arg Glu Pro Tyr Asp Ser
    1430                1435                1440

Val Glu Glu Ser Ser Glu Ser Glu Asn Ser Pro Val Pro Gln Arg
    1445                1450                1455

Lys Arg Arg Thr Ser Val Gly Ser Ser Ser Ser Asp Glu Tyr Lys
    1460                1465                1470

Gln Glu Asp Ser Gln Gly Ser Gly Glu Glu Glu Asp Phe Ile Arg
    1475                1480                1485

Lys Gln Ile Ile Glu Met Ser Ala Asp Glu Asp Ala Ser Gly Ser
    1490                1495                1500

Glu Asp Asp Glu Phe Ile Arg Asn Gln Leu Lys Glu Ile Ser Ser
    1505                1510                1515

Ser Thr Glu Ser Gln Lys Lys Glu Glu Thr Lys Gly Lys Gly Lys
    1520                1525                1530

Ile Thr Ala Gly Lys His Arg Arg Leu Thr Arg Lys Ser Ser Thr
    1535                1540                1545

Ser Ile Asp Glu Asp Ala Gly Arg Arg His Ser Trp His Asp Glu
    1550                1555                1560

Asp Asp Glu Ala Phe Asp Glu Ser Pro Glu Leu Lys Tyr Arg Glu
    1565                1570                1575

Thr Lys Ser Gln Glu Ser Glu Glu Leu Val Val Thr Gly Gly Gly
    1580                1585                1590

Gly Leu Arg Arg Phe Lys Thr Ile Glu Leu Asn Ser Thr Ile Ala
    1595                1600                1605

Asp Lys Tyr Ser Ala Glu Ser Ser Gln Lys Lys Thr Ser Leu Tyr
    1610                1615                1620

Phe Asp Glu Glu Pro Glu Leu Glu Met Glu Ser Leu Thr Asp Ser
    1625                1630                1635

Pro Glu Asp Arg Ser Arg Gly Glu Gly Ser Ser Ser Leu His Ala
    1640                1645                1650

Ser Ser Phe Thr Pro Gly Thr Ser Pro Thr Ser Val Ser Ser Leu
    1655                1660                1665

Asp Glu Asp Ser Asp Ser Ser Pro Ser His Lys Lys Gly Glu Ser
    1670                1675                1680

Lys Gln Gln Arg Lys Ala Arg His Arg Pro His Gly Pro Leu Leu
    1685                1690                1695

Pro Thr Ile Glu Asp Ser Ser Glu Glu Glu Glu Leu Arg Glu Glu
    1700                1705                1710

Glu Glu Leu Leu Lys Glu Gln Glu Lys Gln Arg Glu Ile Glu Gln
    1715                1720                1725

Gln Gln Arg Lys Ser Ser Ser Lys Lys Ser Lys Lys Asp Lys Asp
    1730                1735                1740

Glu Leu Arg Ala Gln Arg Arg Arg Glu Arg Pro Lys Thr Pro Pro
    1745                1750                1755

Ser Asn Leu Ser Pro Ile Glu Asp Ala Ser Pro Thr Glu Glu Leu
    1760                1765                1770

Arg Gln Ala Ala Glu Met Glu Glu Leu His Arg Ser Ser Cys Ser
    1775                1780                1785

Glu Tyr Ser Pro Ser Ile Glu Ser Asp Pro Glu Gly Phe Glu Ile
    1790                1795                1800

Ser Pro Glu Lys Ile Ile Glu Val Gln Lys Val Tyr Lys Leu Pro
    1805                1810                1815

Thr Ala Val Ser Leu Tyr Ser Pro Thr Asp Glu Gln Ser Ile Met
    1820                1825                1830

Gln Lys Glu Gly Ser Gln Lys Ala Leu Lys Ser Ala Glu Glu Met
    1835                1840                1845

Tyr Glu Glu Met Met His Lys Thr His Lys Tyr Lys Ala Phe Pro
    1850                1855                1860

Ala Ala Asn Glu Arg Asp Glu Val Phe Glu Lys Glu Pro Leu Tyr
    1865                1870                1875

Gly Gly Met Leu Ile Glu Asp Tyr Ile Tyr Glu Ser Leu Val Glu
    1880                1885                1890

Asp Thr Tyr Asn Gly Ser Val Asp Gly Ser Leu Leu Thr Arg Gln
    1895                1900                1905

Glu Glu Glu Asn Gly Phe Met Gln Gln Lys Gly Arg Glu Gln Lys
    1910                1915                1920

Ile Arg Leu Ser Glu Gln Ile Tyr Glu Asp Pro Met Gln Lys Ile
    1925                1930                1935

Thr Asp Leu Gln Lys Glu Phe Tyr Glu Leu Glu Ser Leu His Ser
    1940                1945                1950

Val Val Pro Gln Glu Asp Ile Val Ser Ser Ser Phe Ile Ile Pro
    1955                1960                1965

Glu Ser His Glu Ile Val Asp Leu Gly Thr Met Val Thr Ser Thr
    1970                1975                1980

Glu Glu Glu Arg Lys Leu Leu Asp Ala Asp Ala Ala Tyr Glu Glu
    1985                1990                1995

Leu Met Lys Arg Gln Gln Met Gln Leu Thr Pro Gly Ser Ser Pro
    2000                2005                2010

Thr Gln Ala Pro Ile Gly Glu Asp Met Thr Glu Ser Thr Met Asp
    2015                2020                2025

Phe Asp Arg Met Pro Asp Ala Ser Leu Thr Ser Ser Val Leu Ser
    2030                2035                2040

Gly Ala Ser Leu Thr Asp Ser Thr Ser Ser Ala Thr Leu Ser Ile
    2045                2050                2055

Pro Asp Val Lys Ile Thr Gln His Phe Ser Thr Glu Glu Ile Glu
    2060                2065                2070

Asp Glu Tyr Val Thr Asp Tyr Thr Arg Glu Ile Gln Glu Ile Ile
    2075                2080                2085

Ala His  Glu Ser Leu Ile Leu  Thr Tyr Ser Glu Pro  Ser Glu Ser
    2090                     2095                     2100

Ala Thr  Ser Val Pro Pro Ser  Asp Thr Pro Ser Leu  Thr Ser Ser
    2105                     2110                     2115

Val Ser  Ser Val Cys Thr Thr  Asp Ser Ser Ser Pro  Ile Thr Thr
    2120                     2125                     2130

Leu Asp  Ser Ile Thr Thr Val  Tyr Thr Glu Pro Val  Asp Met Ile
    2135                     2140                     2145

Thr Lys  Phe Glu Asp Ser Glu  Glu Ile Ser Ser Ser  Thr Tyr Phe
    2150                     2155                     2160

Pro Gly  Ser Ile Ile Asp Tyr  Pro Glu Glu Ile Ser  Val Ser Leu
    2165                     2170                     2175

Asp Arg  Thr Ala Pro Pro Asp  Gly Arg Ala Ser Ala  Asp His Ile
    2180                     2185                     2190

Val Ile  Ser Leu Ser Asp Met  Ala Ser Ser Ile Ile  Glu Ser Val
    2195                     2200                     2205

Val Pro  Lys Pro Glu Gly Pro  Val Ala Asp Thr Val  Ser Thr Asp
    2210                     2215                     2220

Leu Leu  Ile Ser Glu Lys Asp  Pro Val Lys Lys Ala  Lys Lys Glu
    2225                     2230                     2235

Thr Gly  Asn Gly Ile Ile Leu  Glu Val Leu Glu Ala  Tyr Arg Asp
    2240                     2245                     2250

Lys Lys  Glu Leu Glu Ala Glu  Arg Thr Lys Ser Ser  Leu Ser Glu
    2255                     2260                     2265

Thr Val  Phe Asp His Pro Pro  Ser Ser Val Ile Ala  Leu Pro Met
    2270                     2275                     2280

Lys Glu  Gln Leu Ser Thr Thr  Tyr Phe Thr Ser Gly  Glu Thr Phe
    2285                     2290                     2295

Gly Gln  Glu Lys Pro Ala Ser  Gln Leu Pro Ser Gly  Ser Pro Ser
    2300                     2305                     2310

Val Ser  Ser Leu Pro Ala Lys  Pro Arg Pro Phe Phe  Arg Ser Ser
    2315                     2320                     2325

Ser Leu  Asp Ile Ser Ala Gln  Pro Pro Pro Pro Pro  Pro Pro Pro
    2330                     2335                     2340

Pro Pro Pro Pro Pro Pro Pro Pro Pro Pro Pro Pro Pro Pro Leu
    2345                2350                2355

Pro Pro Pro Thr Ser Pro Lys Pro Thr Ile Leu Pro Lys Lys Lys
    2360                2365                2370

Leu Thr Val Ala Ser Pro Val Thr Thr Ala Thr Pro Leu Phe Asp
    2375                2380                2385

Ala Val Thr Thr Leu Glu Thr Thr Ala Val Leu Arg Ser Asn Gly
    2390                2395                2400

Leu Pro Val Thr Arg Ile Cys Thr Thr Ala Pro Pro Pro Val Pro
    2405                2410                2415

Pro Lys Pro Ser Ser Ile Pro Ser Gly Leu Val Phe Thr His Arg
    2420                2425                2430

Pro Glu Pro Ser Lys Pro Pro Ile Ala Pro Lys Pro Val Ile Pro
    2435                2440                2445

Gln Leu Pro Thr Thr Thr Gln Lys Pro Thr Asp Ile His Pro Lys
    2450                2455                2460

Pro Thr Gly Leu Ser Leu Thr Ser Ser Met Thr Leu Asn Leu Val
    2465                2470                2475

Thr Ser Ala Asp Tyr Lys Leu Pro Ser Pro Thr Ser Pro Leu Ser
    2480                2485                2490

Pro His Ser Asn Lys Ser Ser Pro Arg Phe Ser Lys Ser Leu Thr
    2495                2500                2505

Glu Thr Tyr Val Val Ile Thr Leu Pro Ser Glu Pro Gly Thr Pro
    2510                2515                2520

Thr Asp Ser Ser Ala Ser Gln Ala Ile Thr Ser Trp Pro Leu Gly
    2525                2530                2535

Ser Pro Ser Lys Asp Leu Val Ser Val Glu Pro Val Phe Ser Val
    2540                2545                2550

Val Pro Pro Val Thr Ala Val Glu Ile Pro Ile Ser Ser Glu Gln
    2555                2560                2565

Thr Phe Tyr Ile Ser Gly Ala Leu Gln Thr Phe Ser Ala Thr Pro
    2570                2575                2580

Val Thr Ala Pro Ser Ser Phe Gln Ala Ala Pro Thr Ser Val Thr
    2585                2590                2595

Gln Phe Leu Thr Thr Glu Val Ser Lys Thr Glu Val Ser Ala Thr
    2600                2605                2610

Arg Ser Thr Ala Pro Ser Val Gly Leu Ser Ser Ile Ser Ile Thr
    2615                2620                2625

Ile Pro Pro Glu Pro Leu Ala Leu Asp Asn Ile His Leu Glu Lys
    2630                2635                2640

Pro Gln Tyr Lys Glu Asp Gly Lys Leu Gln Leu Val Gly Asp Val
    2645                2650                2655

Ile Asp Leu Arg Thr Val Pro Lys Val Glu Val Lys Thr Thr Asp
    2660                2665                2670

Lys Cys Ile Asp Leu Ser Ala Ser Thr Met Asp Val Lys Arg Gln
    2675                2680                2685

Ile Thr Ala Asn Glu Val Tyr Gly Lys Gln Ile Ser Ala Val Gln
    2690                2695                2700

Pro Ser Ile Ile Asn Leu Ser Val Thr Ser Ser Ile Val Thr Pro
    2705                2710                2715

Val Ser Leu Ala Thr Glu Thr Val Thr Phe Val Thr Cys Thr Ala
    2720                2725                2730

Ser Ala Ser Tyr Thr Thr Gly Thr Glu Ser Leu Val Gly Ala Glu
    2735                2740                2745

His Ala Met Thr Thr Pro Leu Gln Leu Thr Thr Ser Lys His Ala
    2750                2755                2760

Glu Pro Pro Tyr Arg Ile Pro Ser Asp Gln Val Phe Pro Ile Ala
    2765                2770                2775

Arg Glu Glu Ala Pro Ile Asn Leu Ser Leu Gly Thr Pro Ala His
    2780                2785                2790

Ala Val Thr Leu Ala Ile Thr Lys Pro Val Thr Val Pro Pro Val
    2795                2800                2805

Gly Val Thr Asn Gly Trp Thr Asp Ser Thr Val Ser Gln Gly Ile
    2810                2815                2820

Thr Asp Gly Glu Val Val Asp Leu Ser Thr Thr Lys Ser His Arg
    2825                2830                2835

Thr Val Val Thr Met Asp Glu Ser Thr Ser Ser Val Met Thr Lys
    2840                2845                2850

Ile Ile Glu Asp Glu Lys Pro Val Asp Leu Thr Ala Gly Arg Arg
    2855                2860                2865

Ala Val Cys Cys Asp Val Val Tyr Lys Leu Pro Phe Gly Arg Ser
    2870                2875                2880

Cys Thr Ala Gln Gln Pro Ala Thr Thr Leu Pro Glu Asp Arg Phe
    2885                2890                2895

Gly Tyr Arg Asp Asp His Tyr Gln Tyr Asp Arg Ser Gly Pro Tyr
    2900                2905                2910

Gly Tyr Arg Gly Ile Gly Gly Met Lys Pro Ser Met Ser Asp Thr
    2915                2920                2925

Asn Leu Ala Glu Ala Gly His Phe Phe Tyr Lys Ser Lys Asn Ala
    2930                2935                2940

Phe Asp Tyr Ser Glu Gly Thr Asp Thr Ala Val Asp Leu Thr Ser
    2945                2950                2955

Gly Arg Val Thr Thr Gly Glu Val Met Asp Tyr Ser Ser Lys Thr
    2960                2965                2970

Thr Gly Pro Tyr Pro Glu Thr Arg Gln Val Ile Ser Gly Ala Gly
    2975                2980                2985

Ile Ser Thr Pro Gln Tyr Ser Thr Ala Arg Met Thr Pro Pro Pro
    2990                2995                3000

Gly Pro Gln Tyr Cys Val Gly Ser Val Leu Arg Ser Ser Asn Gly
    3005                3010                3015

Val Val Tyr Ser Ser Val Ala Thr Pro Thr Pro Ser Thr Phe Ala
    3020                3025                3030

Ile Thr Thr Gln Pro Gly Ser Ile Phe Ser Thr Thr Val Arg Asp
    3035                3040                3045

Leu Ser Gly Ile His Thr Ala Asp Ala Val Thr Ser Leu Pro Ala
    3050                3055                3060

Met His His Ser Gln Pro Met Pro Arg Ser Tyr Phe Ile Thr Thr
    3065                3070                3075

Gly Ala Ser Glu Thr Asp Ile Ala Val Thr Gly Ile Asp Ile Ser
    3080                3085                3090

Ala Ser Leu Gln Thr Ile Thr Met Glu Ser Leu Thr Ala Glu Thr
    3095                3100                3105

Ile Asp Ser Val Pro Thr Leu Thr Thr Ala Ser Glu Val Phe Pro
    3110                3115                3120

Glu Val Val Gly Asp Glu Ser Ala Leu Leu Ile Val Pro Glu Glu
    3125                3130                3135

Asp Lys Gln Gln Gln Gln Leu Asp Leu Glu Arg Glu Leu Leu Glu
    3140                3145                3150

Leu Glu Lys Ile Lys Gln Gln Arg Phe Ala Glu Glu Leu Glu Trp
    3155                3160                3165

Glu Arg Gln Glu Ile Gln Arg Phe Arg Glu Gln Glu Lys Ile Met
    3170                3175                3180

Val Gln Lys Lys Leu Glu Glu Leu Gln Ser Met Lys Gln His Leu
    3185                3190                3195

Leu Phe Gln Gln Glu Glu Glu Arg Gln Ala Gln Phe Met Met Arg
    3200                3205                3210

Gln Glu Thr Leu Ala Gln Gln Gln Leu Gln Leu Glu Gln Ile Gln
    3215                3220                3225

Gln Leu Gln Gln Gln Leu His Gln Gln Leu Glu Glu Gln Lys Ile
    3230                3235                3240

Arg Gln Ile Tyr Gln Tyr Asn Tyr Asp Pro Ser Gly Thr Ala Ser
    3245                3250                3255

Pro Gln Thr Thr Thr Glu Gln Ala Ile Leu Glu Gly Gln Tyr Ala
    3260                3265                3270

Ala Leu Glu Gly Ser Gln Phe Trp Ala Thr Glu Asp Ala Thr Thr
    3275                3280                3285

Thr Ala Ser Ala Val Val Ala Ile Glu Ile Pro Gln Ser Gln Gly
    3290                3295                3300

Trp Tyr Thr Val Gln Ser Asp Gly Val Thr Gln Tyr Ile Ala Pro
    3305                3310                3315

Pro Gly Ile Leu Ser Thr Val Ser Glu Ile Pro Leu Thr Asp Val
    3320                3325                3330

Val Val Lys Glu Glu Lys Gln Pro Lys Lys Arg Ser Ser Gly Ala
    3335                3340                3345

Lys Val Arg Gly Gln Tyr Asp Asp Met Gly Glu Asn Met Thr Asp
    3350                3355                3360

Asp Pro Arg Ser Phe Lys Lys Ile Val Asp Ser Gly Val Gln Thr
    3365                3370                3375

Asp Asp Glu Asp Ala Thr Asp Arg Ser Tyr Val Ser Arg Arg Arg
    3380                3385                3390

Arg Thr Lys Lys Ser Val Asp Thr Ser Val Gln Thr Asp Asp Glu
    3395                3400                3405

Asp Gln Asp Glu Trp Asp Met Pro Thr Arg Ser Arg Arg Lys Ala
    3410                3415                3420

Arg Val Gly Lys Tyr Gly Asp Ser Met Thr Glu Ala Asp Lys Thr
    3425                3430                3435

Lys Pro Leu Ser Lys Val Ser Ser Ile Ala Val Gln Thr Val Ala
    3440                3445                3450

Glu Ile Ser Val Gln Thr Glu Pro Val Gly Thr Ile Arg Thr Pro
    3455                3460                3465

Ser Ile Arg Ala Arg Val Asp Ala Lys Val Glu Ile Ile Lys His
    3470                3475                3480

Ile Ser Ala Pro Glu Lys Thr Tyr Lys Gly Gly Ser Leu Gly Cys
    3485                3490                3495

Gln Thr Glu Ala Asp Ser Asp Thr Gln Ser Pro Gln Tyr Leu Ser
    3500                3505                3510

Ala Thr Ser Pro Pro Lys Asp Lys Lys Arg Pro Thr Pro Leu Glu
    3515                3520                3525

Ile Gly Tyr Ser Ser His Leu Arg Ala Asp Ser Thr Val Gln Leu
    3530                3535                3540

Ala Pro Ser Pro Pro Lys Ser Pro Lys Val Leu Tyr Ser Pro Ile
    3545                3550                3555

Ser Pro Leu Ser Pro Gly Lys Ala Leu Glu Ser Ala Phe Val Pro
    3560                3565                3570

Tyr Glu Lys Pro Leu Pro Asp Asp Ile Ser Pro Gln Lys Val Leu
    3575                3580                3585

His Pro Asp Met Ala Lys Val Pro Pro Ala Ser Pro Lys Thr Ala
    3590                3595                3600

Lys Met Met Gln Arg Ser Met Ser Asp Pro Lys Pro Leu Ser Pro
    3605                3610                3615

Thr Ala Asp Glu Ser Ser Arg Ala Pro Phe Gln Tyr Thr Glu Gly
    3620                3625                3630

Tyr Thr Thr Lys Gly Ser Gln Thr Met Thr Ser Ser Gly Ala Gln
    3635                3640                3645

Lys Lys Val Lys Arg Thr Leu Pro Asn Pro Pro Pro Glu Glu Ile
    3650                3655                3660

Ser Thr Gly Thr Gln Ser Thr Phe Ser Thr Met Gly Thr Val Ser
    3665                3670                3675

Arg Arg Arg Ile Cys Arg Thr Asn Thr Met Ala Arg Ala Lys Ile
    3680                3685                3690

Leu Gln Asp Ile Asp Arg Glu Leu Asp Leu Val Glu Arg Glu Ser
    3695                3700                3705

Ala Lys Leu Arg Lys Lys Gln Ala Glu Leu Asp Glu Glu Glu Lys
    3710                3715                3720

Glu Ile Asp Ala Lys Leu Arg Tyr Leu Glu Met Gly Ile Asn Arg
    3725                3730                3735

Arg Lys Glu Ala Leu Leu Lys Glu Arg Glu Lys Arg Glu Arg Ala
    3740                3745                3750

Tyr Leu Gln Gly Val Ala Glu Asp Arg Asp Tyr Met Ser Asp Ser
    3755                3760                3765

Glu Val Ser Ser Thr Arg Pro Thr Arg Ile Glu Ser Gln His Gly
    3770                3775                3780

Ile Glu Arg Pro Arg Thr Ala Pro Gln Thr Glu Phe Ser Gln Phe
    3785                3790                3795

Ile Pro Pro Gln Thr Gln Thr Glu Ser Gln Leu Val Pro Pro Thr
    3800                3805                3810

Ser Pro Tyr Thr Gln Tyr Gln Tyr Ser Ser Pro Ala Leu Pro Thr
    3815                3820                3825

Gln Ala Pro Thr Ser Tyr Thr Gln Gln Ser His Phe Glu Gln Gln
    3830                3835                3840

Thr Leu Tyr His Gln Gln Val Ser Pro Tyr Gln Thr Gln Pro Thr
    3845                3850                3855

Phe Gln Ala Val Ala Thr Met Ser Phe Thr Pro Gln Val Gln Pro
    3860                3865                3870

Thr Pro Thr Pro Gln Pro Ser Tyr Gln Leu Pro Ser Gln Met Met
    3875                3880                3885

Val Ile Gln Gln Lys Pro Arg Gln Thr Thr Leu Tyr Leu Glu Pro
    3890                3895                3900

Lys Ile Thr Ser Asn Tyr Glu Val Ile Arg Asn Gln Pro Leu Met
    3905                3910                3915

Ile Ala Pro Val Ser Thr Asp Asn Thr Phe Ala Val Ser His Leu
    3920                3925                3930

Gly Ser Lys Tyr Asn Ser Leu Asp Leu Arg Ile Gly Leu Glu Glu
    3935                3940                3945

Arg Ser Ser Met Ala Ser Ser Pro Ile Ser Ser Ile Ser Ala Asp
    3950                3955                3960

Ser Phe Tyr Ala Asp Ile Asp His His Thr Pro Arg Asn Tyr Val
    3965                3970                3975

Leu Ile Asp Asp Ile Gly Glu Ile Thr Lys Gly Thr Ala Ala Leu
    3980                3985                3990

Ser Thr Ala Phe Ser Leu His Glu Lys Asp Leu Ser Lys Thr Asp
    3995                4000                4005

Arg Leu Leu Arg Thr Thr Glu Thr Arg Arg Ser Gln Glu Val Thr
    4010                4015                4020

Asp Phe Leu Ala Pro Leu Gln Ser Ser Ser Arg Leu His Ser Tyr
    4025                4030                4035

Val Lys Ala Glu Glu Asp Pro Met Glu Asp Pro Tyr Glu Leu Lys
    4040                4045                4050

Leu Leu Lys His Gln Ile Lys Gln Glu Phe Arg Arg Gly Thr Glu
    4055                4060                4065

Ser Leu Asp His Leu Ala Gly Leu Ser His Tyr Tyr His Ala Asp
    4070                4075                4080

Thr Ser Tyr Arg His Phe Pro Lys Ser Glu Lys Tyr Ser Ile Ser
    4085                4090                4095

Arg Leu Thr Leu Glu Lys Gln Ala Ala Lys Gln Leu Pro Ala Ala
    4100                4105                4110

Ile Leu Tyr Gln Lys Gln Ser Lys His Lys Lys Ser Leu Ile Asp
    4115                4120                4125

Pro Lys Met Ser Lys Phe Ser Pro Ile Gln Glu Ser Arg Asp Leu
    4130                    4135                    4140

Glu Pro Asp Tyr Ser Ser Tyr Met Thr Ser Ser Thr Ser Ser Ile
    4145                    4150                    4155

Gly Gly Ile Ser Ser Arg Ala Arg Leu Leu Gln Asp Asp Ile Thr
    4160                    4165                    4170

Phe Gly Leu Arg Lys Asn Ile Thr Asp Gln Gln Lys Phe Met Gly
    4175                    4180                    4185

Ser Ser Leu Gly Thr Gly Leu Gly Thr Leu Gly Asn Thr Ile Arg
    4190                    4195                    4200

Ser Ala Leu Gln Asp Glu Ala Asp Lys Pro Tyr Ser Ser Gly Ser
    4205                    4210                    4215

Arg Ser Arg Pro Ser Ser Arg Pro Ser Ser Val Tyr Gly Leu Asp
    4220                    4225                    4230

Leu Ser Ile Lys Arg Asp Ser Ser Ser Ser Ser Leu Arg Leu Lys
    4235                    4240                    4245

Ala Gln Glu Ala Glu Ala Leu Asp Val Ser Phe Ser His Ala Ser
    4250                    4255                    4260

Ser Ser Ala Arg Thr Lys Pro Thr Ser Leu Pro Ile Ser Gln Ser
    4265                    4270                    4275

Arg Gly Arg Ile Pro Ile Val Ala Gln Asn Ser Glu Glu Glu Ser
    4280                    4285                    4290

Pro Leu Ser Pro Val Gly Gln Pro Met Gly Met Ala Arg Ala Ala
    4295                    4300                    4305

Ala Gly Pro Leu Pro Pro Ile Ser Ala Asp Thr Arg Asp Gln Phe
    4310                    4315                    4320

Gly Ser Ser His Ser Leu Pro Glu Val Gln Gln His Met Arg Glu
    4325                    4330                    4335

Glu Ser Arg Thr Arg Gly Tyr Asp Arg Asp Ile Ala Phe Ile Met
    4340                    4345                    4350

Asp Asp Phe Gln His Ala Met Ser Asp Ser Glu Ala Tyr His Leu
    4355                    4360                    4365

Arg Arg Glu Glu Thr Asp Trp Phe Asp Lys Pro Arg Glu Ser Arg
    4370                    4375                    4380

Leu Glu Asn Gly His Gly Leu Asp Arg Lys Leu Pro Glu Arg Leu
    4385                4390                4395

Val His Ser Arg Pro Leu Ser Gln His Gln Glu Gln Ile Ile Gln
    4400                4405                4410

Met Asn Gly Lys Thr Met His Tyr Ile Phe Pro His Ala Arg Ile
    4415                4420                4425

Lys Ile Thr Arg Asp Ser Lys Asp His Thr Val Ser Met Gln Val
    4430                4435                4440

Leu Glu Trp Asn Gly Ile Pro Leu Thr Ser Lys Thr Tyr Glu Glu
    4445                4450                4455

Val Gln Ser Ile Ile Ser Gln Gln Ser Gly Glu Ala Glu Ile Cys
    4460                4465                4470

Val Arg Leu Asp Leu Asn Met Leu Ser Asp Ser Glu Asn Ser Gln
    4475                4480                4485

His Leu Glu Leu His Glu Pro Pro Lys Ala Val Asp Lys Ala Lys
    4490                4495                4500

Ser Pro Gly Val Asp Pro Lys Gln Leu Ala Ala Glu Leu Gln Lys
    4505                4510                4515

Val Ser Leu Gln Gln Ser Pro Leu Val Leu Ser Ser Val Val Glu
    4520                4525                4530

Lys Gly Ser His Val His Ser Gly Pro Thr Ser Ala Gly Ser Ser
    4535                4540                4545

Ser Val Pro Ser Pro Gly Gln Pro Gly Ser Pro Ser Val Ser Lys
    4550                4555                4560

Lys Lys His Gly Ser Ser Lys Leu Gln Ile Asn Tyr Asp Leu Gly
    4565                4570                4575

Asn Leu Ile Ile His Ile Leu Gln Ala Arg Asn Leu Val Pro Arg
    4580                4585                4590

Asp Asn Asn Gly Tyr Ser Asp Pro Phe Val Lys Val Tyr Leu Leu
    4595                4600                4605

Pro Gly Arg Gly Ala Glu Tyr Lys Arg Arg Thr Lys His Val Gln
    4610                4615                4620

Lys Ser Leu Asn Pro Glu Trp Asn Gln Thr Val Ile Tyr Lys Ser
    4625                4630                4635

Ile Ser Met Glu Gln Leu Lys Lys Lys Thr Leu Glu Val Thr Val
    4640                    4645                    4650

Trp Asp Tyr Asp Arg Phe Ser Ser Asn Asp Phe Leu Gly Glu Val
    4655                    4660                    4665

Leu Ile Asp Leu Ser Ser Thr Ser His Leu Asp Asn Thr Pro Arg
    4670                    4675                    4680

Trp Tyr Pro Leu Lys Glu Gln Thr Glu Ser Ile Asp His Gly Lys
    4685                    4690                    4695

Ser His Ser Ser Gln Ser Ser Gln Gln Ser Pro Lys Pro Ser Val
    4700                    4705                    4710

Ile Lys Ser Arg Ser His Gly Ile Phe Pro Asp Pro Ser Lys Asp
    4715                    4720                    4725

Met Gln Val Pro Thr Ile Glu Lys Ser His Ser Ser Pro Gly Ser
    4730                    4735                    4740

Ser Lys Ser Ser Ser Glu Gly His Leu Arg Ser His Gly Pro Ser
    4745                    4750                    4755

Arg Ser Gln Ser Lys Thr Ser Val Thr Gln Thr His Leu Glu Asp
    4760                    4765                    4770

Ala Gly Ala Ala Ile Ala Ala Ala Glu Ala Ala Val Gln Gln Leu
    4775                    4780                    4785

Arg Ile Gln Pro Thr Ala His Lys Ser Gly Gln Ser Asn His Ala
    4790                    4795                    4800

Arg Lys Gln His Arg His Ser Ile Ala Gly Val Leu Pro Ile Gln
    4805                    4810                    4815

Arg Thr Gln Ser Asp Asn Leu Pro Pro Pro Ala Asn Gly Asn Gln
    4820                    4825                    4830

Asp Gln Ser Gln Leu Ala Leu Arg Lys Val Met Ser Asp Gly Pro
    4835                    4840                    4845

Val Lys Pro Glu Gly Ala Lys Pro Pro Asn His Arg Pro Ala Glu
    4850                    4855                    4860

Ser Ser Val Ser Thr Gly Ser Ser Gly Ser Ser Phe Gly Ser Gly
    4865                    4870                    4875

Tyr Ser Val Asp Ser Glu Gly Ser Ser Ser Thr Ala Gly Glu Thr
    4880                    4885                    4890

Asn Leu Phe Pro Ile Pro Arg Ile Gly Lys Met Gly Gln Asn Gly
    4895                    4900                    4905

Gln Glu Pro Val Lys Gln Pro Gly Val Gly Val Gly Leu Ala Asp
    4910                    4915                    4920

Thr Glu Ala Arg Gly Gln Arg Glu Pro Lys Pro Gly Gln Ala Cys
    4925                    4930                    4935

Phe Leu Gly Ala Arg Asn Met Lys Glu Ile Gln Pro Met Pro Glu
    4940                    4945                    4950

Ile Tyr Pro Glu Thr Asp Lys Asp Gly Glu Lys Tyr Pro Gly Ser
    4955                    4960                    4965

Leu Phe Leu Pro Phe Ser Asn Leu Cys Gln Cys Leu Ile Lys Thr
    4970                    4975                    4980

Asn Trp Lys Pro Val Asp Gly Glu Ala Gln Ala Asp Leu Glu Leu
    4985                    4990                    4995

Leu Pro Asn Leu Gln Thr Arg Arg Ile Leu Pro Pro Ser Met Tyr
    5000                    5005                    5010

Leu Leu Gln Lys Thr Lys Ala Leu Gln Thr Tyr Lys Arg Ser Asp
    5015                    5020                    5025

Ile Arg Gly Ser Asn Gly Pro Thr Ile Asn Ala Thr Thr Cys Phe
    5030                    5035                    5040

Glu Thr Thr Ser Val Met Gly Glu Ile Lys Ile Ala Leu Lys Lys
    5045                    5050                    5055

Glu Met Lys Thr Asp Gly Glu Gln Leu Ile Val Glu Ile Leu Gln
    5060                    5065                    5070

Cys Arg Asn Ile Thr Tyr Lys Phe Lys Ser Pro Asp His Leu Pro
    5075                    5080                    5085

Asp Leu Tyr Val Lys Ile Tyr Val Met Asn Ile Ser Thr Gln Lys
    5090                    5095                    5100

Lys Val Ile Lys Lys Lys Thr Arg Val Cys Arg His Asp Arg Glu
    5105                    5110                    5115

Pro Ser Phe Asn Glu Thr Phe Arg Phe Ser Leu Ser Pro Ala Gly
    5120                    5125                    5130

His Ser Leu Gln Ile Leu Leu Phe Ser Asn Gly Gly Lys Phe Met
    5135                    5140                    5145

Lys Lys  Thr Leu Ile Gly Glu  Ala Cys Ile Trp Leu  Asp Lys Val
    5150                5155                5160

Asp Leu  Arg Lys Arg Ile Val  Asn Trp His Lys Leu  Leu Val Ser
    5165                5170                5175

Pro Thr  Gln Thr His
    5180

<210>  7
<211>  2386
<212>  PRT
<213>  Homo sapiens

<400>  7

Met Leu Arg Gly Pro Gly Pro Gly Leu Leu Leu Leu Ala Val Gln Cys
1               5                   10                  15

Leu Gly Thr Ala Val Pro Ser Thr Gly Ala Ser Lys Ser Lys Arg Gln
            20                  25                  30

Ala Gln Gln Met Val Gln Pro Gln Ser Pro Val Ala Val Ser Gln Ser
        35                  40                  45

Lys Pro Gly Cys Tyr Asp Asn Gly Lys His Tyr Gln Ile Asn Gln Gln
    50                  55                  60

Trp Glu Arg Thr Tyr Leu Gly Asn Ala Leu Val Cys Thr Cys Tyr Gly
65                  70                  75                  80

Gly Ser Arg Gly Phe Asn Cys Glu Ser Lys Pro Glu Ala Glu Glu Thr
                85                  90                  95

Cys Phe Asp Lys Tyr Thr Gly Asn Thr Tyr Arg Val Gly Asp Thr Tyr
            100                 105                 110

Glu Arg Pro Lys Asp Ser Met Ile Trp Asp Cys Thr Cys Ile Gly Ala
        115                 120                 125

Gly Arg Gly Arg Ile Ser Cys Thr Ile Ala Asn Arg Cys His Glu Gly
    130                 135                 140

Gly Gln Ser Tyr Lys Ile Gly Asp Thr Trp Arg Arg Pro His Glu Thr
145                 150                 155                 160

Gly Gly Tyr Met Leu Glu Cys Val Cys Leu Gly Asn Gly Lys Gly Glu
                165                 170                 175

Trp Thr Cys Lys Pro Ile Ala Glu Lys Cys Phe Asp His Ala Ala Gly
            180                 185                 190

Thr Ser Tyr Val Val Gly Glu Thr Trp Glu Lys Pro Tyr Gln Gly Trp
        195                 200                 205

Met Met Val Asp Cys Thr Cys Leu Gly Glu Gly Ser Gly Arg Ile Thr
    210                 215                 220

Cys Thr Ser Arg Asn Arg Cys Asn Asp Gln Asp Thr Arg Thr Ser Tyr
225                 230                 235                 240

Arg Ile Gly Asp Thr Trp Ser Lys Lys Asp Asn Arg Gly Asn Leu Leu
                245                 250                 255

Gln Cys Ile Cys Thr Gly Asn Gly Arg Gly Glu Trp Lys Cys Glu Arg
            260                 265                 270

His Thr Ser Val Gln Thr Thr Ser Ser Gly Ser Gly Pro Phe Thr Asp
        275                 280                 285

Val Arg Ala Ala Val Tyr Gln Pro Gln Pro His Pro Gln Pro Pro Pro
    290                 295                 300

Tyr Gly His Cys Val Thr Asp Ser Gly Val Val Tyr Ser Val Gly Met
305                 310                 315                 320

Gln Trp Leu Lys Thr Gln Gly Asn Lys Gln Met Leu Cys Thr Cys Leu
                325                 330                 335

Gly Asn Gly Val Ser Cys Gln Glu Thr Ala Val Thr Gln Thr Tyr Gly
            340                 345                 350

Gly Asn Ser Asn Gly Glu Pro Cys Val Leu Pro Phe Thr Tyr Asn Gly
        355                 360                 365

Arg Thr Phe Tyr Ser Cys Thr Thr Glu Gly Arg Gln Asp Gly His Leu
    370                 375                 380

Trp Cys Ser Thr Thr Ser Asn Tyr Glu Gln Asp Gln Lys Tyr Ser Phe
385                 390                 395                 400

Cys Thr Asp His Thr Val Leu Val Gln Thr Gln Gly Gly Asn Ser Asn
                405                 410                 415

Gly Ala Leu Cys His Phe Pro Phe Leu Tyr Asn Asn His Asn Tyr Thr
            420                 425                 430

Asp Cys Thr Ser Glu Gly Arg Arg Asp Asn Met Lys Trp Cys Gly Thr
        435                 440                 445

Thr Gln Asn Tyr Asp Ala Asp Gln Lys Phe Gly Phe Cys Pro Met Ala
    450                 455                 460

Ala His Glu Glu Ile Cys Thr Thr Asn Glu Gly Val Met Tyr Arg Ile
465                 470                 475                 480

Gly Asp Gln Trp Asp Lys Gln His Asp Met Gly His Met Met Arg Cys
                485                 490                 495

Thr Cys Val Gly Asn Gly Arg Gly Glu Trp Thr Cys Ile Ala Tyr Ser
            500                 505                 510

Gln Leu Arg Asp Gln Cys Ile Val Asp Asp Ile Thr Tyr Asn Val Asn
        515                 520                 525

Asp Thr Phe His Lys Arg His Glu Glu Gly His Met Leu Asn Cys Thr
    530                 535                 540

Cys Phe Gly Gln Gly Arg Gly Arg Trp Lys Cys Asp Pro Val Asp Gln
545                 550                 555                 560

Cys Gln Asp Ser Glu Thr Gly Thr Phe Tyr Gln Ile Gly Asp Ser Trp
                565                 570                 575

Glu Lys Tyr Val His Gly Val Arg Tyr Gln Cys Tyr Cys Tyr Gly Arg
            580                 585                 590

Gly Ile Gly Glu Trp His Cys Gln Pro Leu Gln Thr Tyr Pro Ser Ser
        595                 600                 605

Ser Gly Pro Val Glu Val Phe Ile Thr Glu Thr Pro Ser Gln Pro Asn
    610                 615                 620

Ser His Pro Ile Gln Trp Asn Ala Pro Gln Pro Ser His Ile Ser Lys
625                 630                 635                 640

Tyr Ile Leu Arg Trp Arg Pro Lys Asn Ser Val Gly Arg Trp Lys Glu
                645                 650                 655

Ala Thr Ile Pro Gly His Leu Asn Ser Tyr Thr Ile Lys Gly Leu Lys
            660                 665                 670

Pro Gly Val Val Tyr Glu Gly Gln Leu Ile Ser Ile Gln Gln Tyr Gly
        675                 680                 685

His Gln Glu Val Thr Arg Phe Asp Phe Thr Thr Thr Ser Thr Ser Thr
    690                 695                 700

Pro Val Thr Ser Asn Thr Val Thr Gly Glu Thr Thr Pro Phe Ser Pro
705                 710                 715                 720

Leu Val Ala Thr Ser Glu Ser Val Thr Glu Ile Thr Ala Ser Ser Phe
                725                 730                 735

Val Val Ser Trp Val Ser Ala Ser Asp Thr Val Ser Gly Phe Arg Val
            740                 745                 750

Glu Tyr Glu Leu Ser Glu Glu Gly Asp Glu Pro Gln Tyr Leu Asp Leu
        755                 760                 765

Pro Ser Thr Ala Thr Ser Val Asn Ile Pro Asp Leu Leu Pro Gly Arg
    770                 775                 780

Lys Tyr Ile Val Asn Val Tyr Gln Ile Ser Glu Asp Gly Glu Gln Ser
785                 790                 795                 800

Leu Ile Leu Ser Thr Ser Gln Thr Thr Ala Pro Asp Ala Pro Pro Asp
                805                 810                 815

Pro Thr Val Asp Gln Val Asp Asp Thr Ser Ile Val Val Arg Trp Ser
            820                 825                 830

Arg Pro Gln Ala Pro Ile Thr Gly Tyr Arg Ile Val Tyr Ser Pro Ser
        835                 840                 845

Val Glu Gly Ser Ser Thr Glu Leu Asn Leu Pro Glu Thr Ala Asn Ser
    850                 855                 860

Val Thr Leu Ser Asp Leu Gln Pro Gly Val Gln Tyr Asn Ile Thr Ile
865                 870                 875                 880

Tyr Ala Val Glu Glu Asn Gln Glu Ser Thr Pro Val Val Ile Gln Gln
                885                 890                 895

Glu Thr Thr Gly Thr Pro Arg Ser Asp Thr Val Pro Ser Pro Arg Asp
            900                 905                 910

Leu Gln Phe Val Glu Val Thr Asp Val Lys Val Thr Ile Met Trp Thr
        915                 920                 925

Pro Pro Glu Ser Ala Val Thr Gly Tyr Arg Val Asp Val Ile Pro Val
    930                 935                 940

Asn Leu Pro Gly Glu His Gly Gln Arg Leu Pro Ile Ser Arg Asn Thr
945                 950                 955                 960

Phe Ala Glu Val Thr Gly Leu Ser Pro Gly Val Thr Tyr Tyr Phe Lys
                965                 970                 975

Val Phe Ala Val Ser His Gly Arg Glu Ser Lys Pro Leu Thr Ala Gln
            980                 985                 990

Gln Thr Thr Lys Leu Asp Ala Pro Thr Asn Leu Gln Phe Val Asn Glu
        995                 1000                1005

Thr Asp Ser Thr Val Leu Val Arg Trp Thr Pro Pro Arg Ala Gln
    1010                1015                1020

Ile Thr Gly Tyr Arg Leu Thr Val Gly Leu Thr Arg Arg Gly Gln
    1025                1030                1035

Pro Arg Gln Tyr Asn Val Gly Pro Ser Val Ser Lys Tyr Pro Leu
    1040                1045                1050

Arg Asn Leu Gln Pro Ala Ser Glu Tyr Thr Val Ser Leu Val Ala
    1055                1060                1065

Ile Lys Gly Asn Gln Glu Ser Pro Lys Ala Thr Gly Val Phe Thr
    1070                1075                1080

Thr Leu Gln Pro Gly Ser Ser Ile Pro Pro Tyr Asn Thr Glu Val
    1085                1090                1095

Thr Glu Thr Thr Ile Val Ile Thr Trp Thr Pro Ala Pro Arg Ile
    1100                1105                1110

Gly Phe Lys Leu Gly Val Arg Pro Ser Gln Gly Gly Glu Ala Pro
    1115                1120                1125

Arg Glu Val Thr Ser Asp Ser Gly Ser Ile Val Val Ser Gly Leu
    1130                1135                1140

Thr Pro Gly Val Glu Tyr Val Tyr Thr Ile Gln Val Leu Arg Asp
    1145                1150                1155

Gly Gln Glu Arg Asp Ala Pro Ile Val Asn Lys Val Val Thr Pro
    1160                1165                1170

Leu Ser Pro Pro Thr Asn Leu His Leu Glu Ala Asn Pro Asp Thr
    1175                1180                1185

Gly Val Leu Thr Val Ser Trp Glu Arg Ser Thr Thr Pro Asp Ile
    1190                1195                1200

Thr Gly Tyr Arg Ile Thr Thr Thr Pro Thr Asn Gly Gln Gln Gly
    1205                1210                1215

Asn Ser Leu Glu Glu Val Val His Ala Asp Gln Ser Ser Cys Thr
    1220                1225                1230

Phe Asp Asn Leu Ser Pro Gly Leu Glu Tyr Asn Val Ser Val Tyr
    1235                1240                1245

Thr Val Lys Asp Asp Lys Glu Ser Val Pro Ile Ser Asp Thr Ile
    1250                1255                1260

Ile Pro Ala Val Pro Pro Pro Thr Asp Leu Arg Phe Thr Asn Ile
    1265                1270                1275

Gly Pro Asp Thr Met Arg Val Thr Trp Ala Pro Pro Pro Ser Ile
    1280                1285                1290

Asp Leu Thr Asn Phe Leu Val Arg Tyr Ser Pro Val Lys Asn Glu
    1295                1300                1305

Glu Asp Val Ala Glu Leu Ser Ile Ser Pro Ser Asp Asn Ala Val
    1310                1315                1320

Val Leu Thr Asn Leu Leu Pro Gly Thr Glu Tyr Val Val Ser Val
    1325                1330                1335

Ser Ser Val Tyr Glu Gln His Glu Ser Thr Pro Leu Arg Gly Arg
    1340                1345                1350

Gln Lys Thr Gly Leu Asp Ser Pro Thr Gly Ile Asp Phe Ser Asp
    1355                1360                1365

Ile Thr Ala Asn Ser Phe Thr Val His Trp Ile Ala Pro Arg Ala
    1370                1375                1380

Thr Ile Thr Gly Tyr Arg Ile Arg His His Pro Glu His Phe Ser
    1385                1390                1395

Gly Arg Pro Arg Glu Asp Arg Val Pro His Ser Arg Asn Ser Ile
    1400                1405                1410

Thr Leu Thr Asn Leu Thr Pro Gly Thr Glu Tyr Val Val Ser Ile
    1415                1420                1425

Val Ala Leu Asn Gly Arg Glu Glu Ser Pro Leu Leu Ile Gly Gln
    1430                1435                1440

Gln Ser Thr Val Ser Asp Val Pro Arg Asp Leu Glu Val Val Ala
    1445                1450                1455

Ala Thr Pro Thr Ser Leu Leu Ile Ser Trp Asp Ala Pro Ala Val
    1460                1465                1470

Thr Val Arg Tyr Tyr Arg Ile Thr Tyr Gly Glu Thr Gly Gly Asn
    1475                1480                1485

Ser Pro Val Gln Glu Phe Thr Val Pro Gly Ser Lys Ser Thr Ala
    1490                1495                1500

Thr Ile Ser Gly Leu Lys Pro Gly Val Asp Tyr Thr Ile Thr Val
    1505                1510                1515

Tyr Ala Val Thr Gly Arg Gly Asp Ser Pro Ala Ser Ser Lys Pro
    1520                1525                1530

Ile Ser Ile Asn Tyr Arg Thr Glu Ile Asp Lys Pro Ser Gln Met
    1535                1540                1545

Gln Val Thr Asp Val Gln Asp Asn Ser Ile Ser Val Lys Trp Leu
    1550                1555                1560

Pro Ser Ser Ser Pro Val Thr Gly Tyr Arg Val Thr Thr Thr Pro
    1565                1570                1575

Lys Asn Gly Pro Gly Pro Thr Lys Thr Lys Thr Ala Gly Pro Asp
    1580                1585                1590

Gln Thr Glu Met Thr Ile Glu Gly Leu Gln Pro Thr Val Glu Tyr
    1595                1600                1605

Val Val Ser Val Tyr Ala Gln Asn Pro Ser Gly Glu Ser Gln Pro
    1610                1615                1620

Leu Val Gln Thr Ala Val Thr Asn Ile Asp Arg Pro Lys Gly Leu
    1625                1630                1635

Ala Phe Thr Asp Val Asp Val Asp Ser Ile Lys Ile Ala Trp Glu
    1640                1645                1650

Ser Pro Gln Gly Gln Val Ser Arg Tyr Arg Val Thr Tyr Ser Ser
    1655                1660                1665

Pro Glu Asp Gly Ile His Glu Leu Phe Pro Ala Pro Asp Gly Glu
    1670                1675                1680

Glu Asp Thr Ala Glu Leu Gln Gly Leu Arg Pro Gly Ser Glu Tyr
    1685                1690                1695

Thr Val Ser Val Val Ala Leu His Asp Asp Met Glu Ser Gln Pro
    1700                1705                1710

Leu Ile Gly Thr Gln Ser Thr Ala Ile Pro Ala Pro Thr Asp Leu
    1715                1720                1725

Lys Phe Thr Gln Val Thr Pro Thr Ser Leu Ser Ala Gln Trp Thr
    1730                1735                1740

Pro Pro Asn Val Gln Leu Thr Gly Tyr Arg Val Arg Val Thr Pro
    1745                1750                1755

Lys Glu Lys Thr Gly Pro Met Lys Glu Ile Asn Leu Ala Pro Asp
    1760                1765                1770

Ser Ser Ser Val Val Val Ser Gly Leu Met Val Ala Thr Lys Tyr
    1775                1780                1785

Glu Val Ser Val Tyr Ala Leu Lys Asp Thr Leu Thr Ser Arg Pro
    1790                1795                1800

Ala Gln Gly Val Val Thr Thr Leu Glu Asn Val Ser Pro Pro Arg
    1805                1810                1815

Arg Ala Arg Val Thr Asp Ala Thr Glu Thr Thr Ile Thr Ile Ser
    1820                1825                1830

Trp Arg Thr Lys Thr Glu Thr Ile Thr Gly Phe Gln Val Asp Ala
    1835                1840                1845

Val Pro Ala Asn Gly Gln Thr Pro Ile Gln Arg Thr Ile Lys Pro
    1850                1855                1860

Asp Val Arg Ser Tyr Thr Ile Thr Gly Leu Gln Pro Gly Thr Asp
    1865                1870                1875

Tyr Lys Ile Tyr Leu Tyr Thr Leu Asn Asp Asn Ala Arg Ser Ser
    1880                1885                1890

Pro Val Val Ile Asp Ala Ser Thr Ala Ile Asp Ala Pro Ser Asn
    1895                1900                1905

Leu Arg Phe Leu Ala Thr Thr Pro Asn Ser Leu Leu Val Ser Trp
    1910                1915                1920

Gln Pro Pro Arg Ala Arg Ile Thr Gly Tyr Ile Ile Lys Tyr Glu
    1925                1930                1935

Lys Pro Gly Ser Pro Pro Arg Glu Val Val Pro Arg Pro Arg Pro
    1940                1945                1950

Gly Val Thr Glu Ala Thr Ile Thr Gly Leu Glu Pro Gly Thr Glu
    1955                1960                1965

Tyr Thr Ile Tyr Val Ile Ala Leu Lys Asn Asn Gln Lys Ser Glu
    1970                1975                1980

Pro Leu Ile Gly Arg Lys Lys Thr Asp Glu Leu Pro Gln Leu Val
    1985                1990                1995

Thr Leu Pro His Pro Asn Leu His Gly Pro Glu Ile Leu Asp Val
    2000                2005                2010

Pro Ser Thr Val Gln Lys Thr Pro Phe Val Thr His Pro Gly Tyr
    2015                2020                2025

Asp Thr Gly Asn Gly Ile Gln Leu Pro Gly Thr Ser Gly Gln Gln
    2030                2035                2040

Pro Ser Val Gly Gln Gln Met Ile Phe Glu Glu His Gly Phe Arg
    2045                2050                2055

Arg Thr Thr Pro Pro Thr Thr Ala Thr Pro Ile Arg His Arg Pro
    2060                2065                2070

Arg Pro Tyr Pro Pro Asn Val Gly Glu Glu Ile Gln Ile Gly His
    2075                2080                2085

Ile Pro Arg Glu Asp Val Asp Tyr His Leu Tyr Pro His Gly Pro
    2090                2095                2100

Gly Leu Asn Pro Asn Ala Ser Thr Gly Gln Glu Ala Leu Ser Gln
    2105                2110                2115

Thr Thr Ile Ser Trp Ala Pro Phe Gln Asp Thr Ser Glu Tyr Ile
    2120                2125                2130

Ile Ser Cys His Pro Val Gly Thr Asp Glu Glu Pro Leu Gln Phe
    2135                2140                2145

Arg Val Pro Gly Thr Ser Thr Ser Ala Thr Leu Thr Gly Leu Thr
    2150                2155                2160

Arg Gly Ala Thr Tyr Asn Ile Ile Val Glu Ala Leu Lys Asp Gln
    2165                2170                2175

Gln Arg His Lys Val Arg Glu Glu Val Val Thr Val Gly Asn Ser
    2180                2185                2190

Val Asn Glu Gly Leu Asn Gln Pro Thr Asp Asp Ser Cys Phe Asp
    2195                2200                2205

Pro Tyr Thr Val Ser His Tyr Ala Val Gly Asp Glu Trp Glu Arg
    2210                2215                2220

Met Ser Glu Ser Gly Phe Lys Leu Leu Cys Gln Cys Leu Gly Phe
    2225                2230                2235

Gly Ser Gly His Phe Arg Cys Asp Ser Ser Arg Trp Cys His Asp
    2240                2245                2250

Asn Gly Val Asn Tyr Lys Ile Gly Glu Lys Trp Asp Arg Gln Gly
    2255                2260                2265

Glu Asn Gly Gln Met Met Ser Cys Thr Cys Leu Gly Asn Gly Lys
    2270                2275                2280

Gly Glu Phe Lys Cys Asp Pro His Glu Ala Thr Cys Tyr Asp Asp
    2285                2290                2295

Gly Lys Thr Tyr His Val Gly Glu Gln Trp Gln Lys Glu Tyr Leu
    2300                2305                2310

Gly Ala Ile Cys Ser Cys Thr Cys Phe Gly Gly Gln Arg Gly Trp
    2315                2320                2325

Arg Cys Asp Asn Cys Arg Arg Pro Gly Gly Glu Pro Ser Pro Glu
    2330                2335                2340

Gly Thr Thr Gly Gln Ser Tyr Asn Gln Tyr Ser Gln Arg Tyr His
    2345                2350                2355

Gln Arg Thr Asn Thr Asn Val Asn Cys Pro Ile Glu Cys Phe Met
    2360                2365                2370

Pro Leu Asp Val Gln Ala Asp Arg Glu Asp Ser Arg Glu
    2375                2380                2385

<210> 8
<211> 314
<212> PRT
<213> Homo sapiens

<400> 8

Met Arg Ile Ala Val Ile Cys Phe Cys Leu Leu Gly Ile Thr Cys Ala
1               5                   10                  15

Ile Pro Val Lys Gln Ala Asp Ser Gly Ser Ser Glu Glu Lys Gln Leu
            20                  25                  30

Tyr Asn Lys Tyr Pro Asp Ala Val Ala Thr Trp Leu Asn Pro Asp Pro
        35                  40                  45

Ser Gln Lys Gln Asn Leu Leu Ala Pro Gln Asn Ala Val Ser Ser Glu
    50                  55                  60

Glu Thr Asn Asp Phe Lys Gln Glu Thr Leu Pro Ser Lys Ser Asn Glu
65                  70                  75                  80

Ser His Asp His Met Asp Asp Met Asp Asp Glu Asp Asp Asp Asp His
                85                  90                  95

Val Asp Ser Gln Asp Ser Ile Asp Ser Asn Asp Ser Asp Asp Val Asp
            100                 105                 110

Asp Thr Asp Asp Ser His Gln Ser Asp Glu Ser His His Ser Asp Glu
        115                 120                 125

Ser Asp Glu Leu Val Thr Asp Phe Pro Thr Asp Leu Pro Ala Thr Glu

130                 135                 140

Val Phe Thr Pro Val Val Pro Thr Val Asp Thr Tyr Asp Gly Arg Gly
145                 150                 155                 160

Asp Ser Val Val Tyr Gly Leu Arg Ser Lys Ser Lys Lys Phe Arg Arg
                165                 170                 175

Pro Asp Ile Gln Tyr Pro Asp Ala Thr Asp Glu Asp Ile Thr Ser His
            180                 185                 190

Met Glu Ser Glu Glu Leu Asn Gly Ala Tyr Lys Ala Ile Pro Val Ala
        195                 200                 205

Gln Asp Leu Asn Ala Pro Ser Asp Trp Asp Ser Arg Gly Lys Asp Ser
    210                 215                 220

Tyr Glu Thr Ser Gln Leu Asp Asp Gln Ser Ala Glu Thr His Ser His
225                 230                 235                 240

Lys Gln Ser Arg Leu Tyr Lys Arg Lys Ala Asn Asp Glu Ser Asn Glu
                245                 250                 255

His Ser Asp Val Ile Asp Ser Gln Glu Leu Ser Lys Val Ser Arg Glu
            260                 265                 270

Phe His Ser His Glu Phe His Ser His Glu Asp Met Leu Val Val Asp
        275                 280                 285

Pro Lys Ser Lys Glu Glu Asp Lys His Leu Lys Phe Arg Ile Ser His
    290                 295                 300

Glu Leu Asp Ser Ala Ser Ser Glu Val Asn
305                 310

<210>  9
<211>  198
<212>  PRT
<213>  Homo sapiens

<400>  9

Met Pro Leu Gly Leu Leu Trp Leu Gly Leu Ala Leu Leu Gly Ala Leu
1               5                   10                  15

His Ala Gln Ala Gln Asp Ser Thr Ser Asp Leu Ile Pro Ala Pro Pro
            20                  25                  30

Leu Ser Lys Val Pro Leu Gln Gln Asn Phe Gln Asp Asn Gln Phe Gln
        35                  40                  45

Gly Lys Trp Tyr Val Val Gly Leu Ala Gly Asn Ala Ile Leu Arg Glu
    50                  55                  60

Asp Lys Asp Pro Gln Lys Met Tyr Ala Thr Ile Tyr Glu Leu Lys Glu
65                  70                  75                  80

Asp Lys Ser Tyr Asn Val Thr Ser Val Leu Phe Arg Lys Lys Lys Cys
                85                  90                  95

Asp Tyr Trp Ile Arg Thr Phe Val Pro Gly Cys Gln Pro Gly Glu Phe
            100                 105                 110

Thr Leu Gly Asn Ile Lys Ser Tyr Pro Gly Leu Thr Ser Tyr Leu Val
        115                 120                 125

Arg Val Val Ser Thr Asn Tyr Asn Gln His Ala Met Val Phe Phe Lys
    130                 135                 140

Lys Val Ser Gln Asn Arg Glu Tyr Phe Lys Ile Thr Leu Tyr Gly Arg
145                 150                 155                 160

Thr Lys Glu Leu Thr Ser Glu Leu Lys Glu Asn Phe Ile Arg Phe Ser
                165                 170                 175

Lys Ser Leu Gly Leu Pro Glu Asn His Ile Val Phe Pro Val Pro Ile
            180                 185                 190

Asp Gln Cys Ile Asp Gly
        195

<210>  10
<211>  457
<212>  PRT
<213>  Homo sapiens

<400>  10

Met Asp Ser Phe Lys Val Val Leu Glu Gly Pro Ala Pro Trp Gly Phe
1               5                   10                  15

Arg Leu Gln Gly Gly Lys Asp Phe Asn Val Pro Leu Ser Ile Ser Arg
            20                  25                  30

Leu Thr Pro Gly Gly Lys Ala Ala Gln Ala Gly Val Ala Val Gly Asp
        35                  40                  45

Trp Val Leu Ser Ile Asp Gly Glu Asn Ala Gly Ser Leu Thr His Ile
    50                  55                  60

Glu Ala Gln Asn Lys Ile Arg Ala Cys Gly Glu Arg Leu Ser Leu Gly
65                  70                  75                  80

Leu Ser Arg Ala Gln Pro Val Gln Ser Lys Pro Gln Lys Ala Ser Ala
                85                  90                  95

Pro Ala Ala Asp Pro Pro Arg Tyr Thr Phe Ala Pro Ser Val Ser Leu
            100                 105                 110

Asn Lys Thr Ala Arg Pro Phe Gly Ala Pro Pro Pro Ala Asp Ser Ala
        115                 120                 125

Pro Gln Gln Asn Gly Gln Pro Leu Arg Pro Leu Val Pro Asp Ala Ser
    130                 135                 140

Lys Gln Arg Leu Met Glu Asn Thr Glu Asp Trp Arg Pro Arg Pro Gly
145                 150                 155                 160

Thr Gly Gln Ser Arg Ser Phe Arg Ile Leu Ala His Leu Thr Gly Thr
                165                 170                 175

Glu Phe Met Gln Asp Pro Asp Glu Glu His Leu Lys Lys Ser Ser Gln
            180                 185                 190

Val Pro Arg Thr Glu Ala Pro Ala Pro Ala Ser Ser Thr Pro Gln Glu
        195                 200                 205

Pro Trp Pro Gly Pro Thr Ala Pro Ser Pro Thr Ser Arg Pro Pro Trp
    210                 215                 220

Ala Val Asp Pro Ala Phe Ala Glu Arg Tyr Ala Pro Asp Lys Thr Ser
225                 230                 235                 240

Thr Val Leu Thr Arg His Ser Gln Pro Ala Thr Pro Thr Pro Leu Gln
                245                 250                 255

Ser Arg Thr Ser Ile Val Gln Ala Ala Ala Gly Gly Val Pro Gly Gly
            260                 265                 270

Gly Ser Asn Asn Gly Lys Thr Pro Val Cys His Gln Cys His Lys Val
        275                 280                 285

Ile Arg Gly Arg Tyr Leu Val Ala Leu Gly His Ala Tyr His Pro Glu
    290                 295                 300

Glu Phe Val Cys Ser Gln Cys Gly Lys Val Leu Glu Glu Gly Gly Phe
305                 310                 315                 320

Phe Glu Glu Lys Gly Ala Ile Phe Cys Pro Pro Cys Tyr Asp Val Arg
                325                 330                 335

Tyr Ala Pro Ser Cys Ala Lys Cys Lys Lys Lys Ile Thr Gly Glu Ile
            340                 345                 350

Met His Ala Leu Lys Met Thr Trp His Val His Cys Phe Thr Cys Ala
        355                 360                 365

Ala Cys Lys Thr Pro Ile Arg Asn Arg Ala Phe Tyr Met Glu Glu Gly
    370                 375                 380

Val Pro Tyr Cys Glu Arg Asp Tyr Glu Lys Met Phe Gly Thr Lys Cys
385                 390                 395                 400

His Gly Cys Asp Phe Lys Ile Asp Ala Gly Asp Arg Phe Leu Glu Ala
                405                 410                 415

Leu Gly Phe Ser Trp His Asp Thr Cys Phe Val Cys Ala Ile Cys Gln
            420                 425                 430

Ile Asn Leu Glu Gly Lys Thr Phe Tyr Ser Lys Lys Asp Arg Pro Leu
        435                 440                 445

Cys Lys Ser His Ala Phe Ser His Val
    450                 455

<210>  11
<211>  638
<212>  PRT
<213>  Homo sapiens

<400>  11

Met Ser Asp Lys Ser Glu Leu Lys Ala Glu Leu Glu Arg Lys Lys Gln
1               5                   10                  15

Arg Leu Ala Gln Ile Arg Glu Glu Lys Lys Arg Lys Glu Glu Glu Arg
            20                  25                  30

Lys Lys Lys Glu Thr Asp Gln Lys Lys Glu Ala Val Ala Pro Val Gln
        35                  40                  45

Glu Glu Ser Asp Leu Glu Lys Lys Arg Arg Glu Ala Glu Ala Leu Leu
    50                  55                  60

Gln Ser Met Gly Leu Thr Pro Glu Ser Pro Ile Val Phe Ser Glu Tyr
65                  70                  75                  80

Trp Val Pro Pro Pro Met Ser Pro Ser Ser Lys Ser Val Ser Thr Pro
                85                  90                  95

Ser Glu Ala Gly Ser Gln Asp Ser Gly Asp Gly Ala Val Gly Ser Arg
            100                 105                 110

Thr Leu His Trp Asp Thr Asp Pro Ser Val Leu Gln Leu His Ser Asp
        115                 120                 125

Ser Asp Leu Gly Arg Gly Pro Ile Lys Leu Gly Met Ala Lys Ile Thr
    130                 135                 140

Gln Val Asp Phe Pro Pro Arg Glu Ile Val Thr Tyr Thr Lys Glu Thr
145                 150                 155                 160

Gln Thr Pro Val Met Ala Gln Pro Lys Glu Asp Glu Glu Glu Asp Asp
                165                 170                 175

Asp Val Val Ala Pro Lys Pro Pro Ile Glu Pro Glu Glu Glu Lys Thr
            180                 185                 190

Leu Lys Lys Asp Glu Glu Asn Asp Ser Lys Ala Pro Pro His Glu Leu
        195                 200                 205

Thr Glu Glu Glu Lys Gln Gln Ile Leu His Ser Glu Glu Phe Leu Ser
    210                 215                 220

Phe Phe Asp His Ser Thr Arg Ile Val Glu Arg Ala Leu Ser Glu Gln
225                 230                 235                 240

Ile Asn Ile Phe Phe Asp Tyr Ser Gly Arg Asp Leu Glu Asp Lys Glu
                245                 250                 255

Gly Glu Ile Gln Ala Gly Ala Lys Leu Ser Leu Asn Arg Gln Phe Phe
            260                 265                 270

Asp Glu Arg Trp Ser Lys His Arg Val Val Ser Cys Leu Asp Trp Ser
        275                 280                 285

Ser Gln Tyr Pro Glu Leu Leu Val Ala Ser Tyr Asn Asn Asn Glu Asp
    290                 295                 300

Ala Pro His Glu Pro Asp Gly Val Ala Leu Val Trp Asn Met Lys Tyr
305                 310                 315                 320

Lys Lys Thr Thr Pro Glu Tyr Val Phe His Cys Gln Ser Ala Val Met
                325                 330                 335

Ser Ala Thr Phe Ala Lys Phe His Pro Asn Leu Val Val Gly Gly Thr
            340                 345                 350

Tyr Ser Gly Gln Ile Val Leu Trp Asp Asn Arg Ser Asn Lys Arg Thr
        355                 360                 365

Pro Val Gln Arg Thr Pro Leu Ser Ala Ala Ala His Thr His Pro Val
    370                 375                 380

Tyr Cys Val Asn Val Val Gly Thr Gln Asn Ala His Asn Leu Ile Ser
385                 390                 395                 400

Ile Ser Thr Asp Gly Lys Ile Cys Ser Trp Ser Leu Asp Met Leu Ser
                405                 410                 415

His Pro Gln Asp Ser Met Glu Leu Val His Lys Gln Ser Lys Ala Val
            420                 425                 430

Ala Val Thr Ser Met Ser Phe Pro Val Gly Asp Val Asn Asn Phe Val
        435                 440                 445

Val Gly Ser Glu Glu Gly Ser Val Tyr Thr Ala Cys Arg His Gly Ser
    450                 455                 460

Lys Ala Gly Ile Ser Glu Met Phe Glu Gly His Gln Gly Pro Ile Thr
465                 470                 475                 480

Gly Ile His Cys His Ala Ala Val Gly Ala Val Asp Phe Ser His Leu
                485                 490                 495

Phe Val Thr Ser Ser Phe Asp Trp Thr Val Lys Leu Trp Thr Thr Lys
            500                 505                 510

Asn Asn Lys Pro Leu Tyr Ser Phe Glu Asp Asn Ala Asp Tyr Val Tyr
        515                 520                 525

Asp Val Met Trp Ser Pro Thr His Pro Ala Leu Phe Ala Cys Val Asp
    530                 535                 540

Gly Met Gly Arg Leu Asp Leu Trp Asn Leu Asn Asn Asp Thr Glu Val
545                 550                 555                 560

Pro Thr Ala Ser Ile Ser Val Glu Gly Asn Pro Ala Leu Asn Arg Val
                565                 570                 575

Arg Trp Thr His Ser Gly Arg Glu Ile Ala Val Gly Asp Ser Glu Gly
            580                 585                 590

Gln Ile Val Ile Tyr Asp Val Gly Glu Gln Ile Ala Val Pro Arg Asn
        595                 600                 605

Asp Glu Trp Ala Arg Phe Gly Arg Thr Leu Ala Glu Ile Asn Ala Asn
    610                 615                 620

Arg Ala Asp Ala Glu Glu Glu Ala Ala Thr Arg Ile Pro Ala
625                 630                 635

<210>  12
<211>  115
<212>  PRT
<213>  Homo sapiens

<400>  12

Met Asn Ala Phe Leu Leu Phe Ala Leu Cys Leu Leu Gly Ala Trp Ala
1               5                   10                  15

Ala Leu Ala Gly Gly Val Thr Val Gln Asp Gly Asn Phe Ser Phe Ser

20                          25                          30

Leu Glu Ser Val Lys Lys Leu Lys Asp Leu Gln Glu Pro Gln Glu Pro
        35                  40                  45

Arg Val Gly Lys Leu Arg Asn Phe Ala Pro Ile Pro Gly Glu Pro Val
    50                  55                  60

Val Pro Ile Leu Cys Ser Asn Pro Asn Phe Pro Glu Glu Leu Lys Pro
65                  70                  75                  80

Leu Cys Lys Glu Pro Asn Ala Gln Glu Ile Leu Gln Arg Leu Glu Glu
                85                  90                  95

Ile Ala Glu Asp Pro Gly Thr Cys Glu Ile Cys Ala Tyr Ala Ala Cys
            100                 105                 110

Thr Gly Cys
        115

<210>  13
<211>  894
<212>  PRT
<213>  Homo sapiens

<400>  13

<210>  14
<211>  1887
<212>  PRT
<213>  Homo sapiens

<400>  14

Met Ala Ala Arg Gly Arg Gly Leu Leu Leu Leu Thr Leu Ser Val Leu
1               5                   10                  15

Leu Ala Ala Gly Pro Ser Ala Ala Ala Ala Lys Leu Asn Ile Pro Lys
            20                  25                  30

Val Leu Leu Pro Phe Thr Arg Ala Thr Arg Val Asn Phe Thr Leu Glu
        35                  40                  45

Ala Ser Glu Gly Cys Tyr Arg Trp Leu Ser Thr Arg Pro Glu Val Ala
    50                  55                  60

Ser Ile Glu Pro Leu Gly Leu Asp Glu Gln Gln Cys Ser Gln Lys Ala
65                  70                  75                  80

Val Val Gln Ala Arg Leu Thr Gln Pro Ala Arg Leu Thr Ser Ile Ile
                85                  90                  95

Phe Ala Glu Asp Ile Thr Thr Gly Gln Val Leu Arg Cys Asp Ala Ile
            100                 105                 110

Val Asp Leu Ile His Asp Ile Gln Ile Val Ser Thr Thr Arg Glu Leu
        115                 120                 125

Tyr Leu Glu Asp Ser Pro Leu Glu Leu Lys Ile Gln Ala Leu Asp Ser
    130                 135                 140

Glu Gly Asn Thr Phe Ser Thr Leu Ala Gly Leu Val Phe Glu Trp Thr
145                 150                 155                 160

Ile Val Lys Asp Ser Glu Ala Asp Arg Phe Ser Asp Ser His Asn Ala
                165                 170                 175

Leu Arg Ile Leu Thr Phe Leu Glu Ser Thr Tyr Ile Pro Pro Ser Tyr
            180                 185                 190

Ile Ser Glu Met Glu Lys Ala Ala Lys Gln Gly Asp Thr Ile Leu Val
        195                 200                 205

Ser Gly Met Lys Thr Gly Ser Ser Lys Leu Lys Ala Arg Ile Gln Glu
    210                 215                 220

Ala Val Tyr Lys Asn Val Arg Pro Ala Glu Val Arg Leu Leu Ile Leu
225                 230                 235                 240

Glu Asn Ile Leu Leu Asn Pro Ala Tyr Asp Val Tyr Leu Met Val Gly
                245                 250                 255

Thr Ser Ile His Tyr Lys Val Gln Lys Ile Arg Gln Gly Lys Ile Thr
            260                 265                 270

Glu Leu Ser Met Pro Ser Asp Gln Tyr Glu Leu Gln Leu Gln Asn Ser
        275                 280                 285

Ile Pro Gly Pro Glu Gly Asp Pro Ala Arg Pro Val Ala Val Leu Ala
    290                 295                 300

Gln Asp Thr Ser Met Val Thr Ala Leu Gln Leu Gly Gln Ser Ser Leu
305                 310                 315                 320

Val Leu Gly His Arg Ser Ile Arg Met Gln Gly Ala Ser Arg Leu Pro
                325                 330                 335

Asn Ser Thr Ile Tyr Val Val Glu Pro Gly Tyr Leu Gly Phe Thr Val
            340                 345                 350

His Pro Gly Asp Arg Trp Val Leu Glu Thr Gly Arg Leu Tyr Glu Ile
        355                 360                 365

Thr Ile Glu Val Phe Asp Lys Phe Ser Asn Lys Val Tyr Val Ser Asp
    370                 375                 380

Asn Ile Arg Ile Glu Thr Val Leu Pro Ala Glu Phe Phe Glu Val Leu
385                 390                 395                 400

Ser Ser Ser Gln Asn Gly Ser Tyr His Arg Ile Arg Ala Leu Lys Arg
                405                 410                 415

Gly Gln Thr Ala Ile Asp Ala Ala Leu Thr Ser Val Val Asp Gln Asp
            420                 425                 430

Gly Gly Val His Ile Leu Gln Val Pro Val Trp Asn Gln Gln Glu Val
        435                 440                 445

Glu Ile His Ile Pro Ile Thr Leu Tyr Pro Ser Ile Leu Thr Phe Pro
    450                 455                 460

Trp Gln Pro Lys Thr Gly Ala Tyr Gln Tyr Thr Ile Arg Ala His Gly
465                 470                 475                 480

Gly Ser Gly Asn Phe Ser Trp Ser Ser Ser Ser His Leu Val Ala Thr
                485                 490                 495

Val Thr Val Lys Gly Val Met Thr Thr Gly Ser Asp Ile Gly Phe Ser
            500                 505                 510

Val Ile Gln Ala His Asp Val Gln Asn Pro Leu His Phe Gly Glu Met
        515                 520                 525

Lys Val Tyr Val Ile Glu Pro His Ser Met Glu Phe Ala Pro Cys Gln
    530                 535                 540

Val Glu Ala Arg Val Gly Gln Ala Leu Glu Leu Pro Leu Arg Ile Ser
545                 550                 555                 560

Gly Leu Met Pro Gly Gly Ala Ser Glu Val Val Thr Leu Ser Asp Cys
                565                 570                 575

Ser His Phe Asp Leu Ala Val Glu Val Glu Asn Gln Gly Val Phe Gln
            580                 585                 590

Pro Leu Pro Gly Arg Leu Pro Pro Gly Ser Glu His Cys Ser Gly Ile
        595                 600                 605

Arg Val Lys Ala Glu Ala Gln Gly Ser Thr Thr Leu Leu Val Ser Tyr
    610                 615                 620

Arg His Gly His Val His Leu Ser Ala Lys Ile Thr Ile Ala Ala Tyr
625                 630                 635                 640

Leu Pro Leu Lys Ala Val Asp Pro Ser Ser Val Ala Leu Val Thr Leu
                645                 650                 655

Gly Ser Ser Lys Glu Met Leu Phe Glu Gly Gly Pro Arg Pro Trp Ile
            660                 665                 670

Leu Glu Pro Ser Lys Phe Phe Gln Asn Val Thr Ala Glu Asp Thr Asp
        675                 680                 685

Ser Ile Gly Leu Ala Leu Phe Ala Pro His Ser Ser Arg Asn Tyr Gln
    690                 695                 700

Gln His Trp Ile Leu Val Thr Cys Gln Ala Leu Gly Glu Gln Val Ile
705                 710                 715                 720

Ala Leu Ser Val Gly Asn Lys Pro Ser Leu Thr Asn Pro Phe Pro Ala
                725                 730                 735

Val Glu Pro Ala Val Val Lys Phe Val Cys Ala Pro Pro Ser Arg Leu
            740                 745                 750

Thr Leu Ala Pro Val Tyr Thr Ser Pro Gln Leu Asp Met Ser Cys Pro
        755                 760                 765

Leu Leu Gln Gln Asn Lys Gln Val Val Pro Val Ser Ser His Arg Asn
    770                 775                 780

Pro Arg Leu Asp Leu Ala Ala Tyr Asp Gln Glu Gly Arg Arg Phe Asp
785                 790                 795                 800

Asn Phe Ser Ser Leu Ser Ile Gln Trp Glu Ser Thr Arg Pro Val Leu
                805                 810                 815

Ala Ser Ile Glu Pro Glu Leu Pro Met Gln Leu Val Ser Gln Asp Asp
            820                 825                 830

Glu Ser Gly Gln Lys Lys Leu His Gly Leu Gln Ala Ile Leu Val His
        835                 840                 845

Glu Ala Ser Gly Thr Thr Ala Ile Thr Ala Thr Ala Thr Gly Tyr Gln
    850                 855                 860

Glu Ser His Leu Ser Ser Ala Arg Thr Lys Gln Pro His Asp Pro Leu
865                 870                 875                 880

Val Pro Leu Ser Ala Ser Ile Glu Leu Ile Leu Val Glu Asp Val Arg
                885                 890                 895

Val Ser Pro Glu Glu Val Thr Ile Tyr Asn His Pro Gly Ile Gln Ala
            900                 905                 910

Glu Leu Arg Ile Arg Glu Gly Ser Gly Tyr Phe Phe Leu Asn Thr Ser
        915                 920                 925

Thr Ala Asp Val Val Lys Val Ala Tyr Gln Glu Ala Arg Gly Val Ala
    930                 935                 940

Met Val His Pro Leu Leu Pro Gly Ser Ser Thr Ile Met Ile His Asp
945                 950                 955                 960

Leu Cys Leu Val Phe Pro Ala Pro Ala Lys Ala Val Val Tyr Val Ser
                965                 970                 975

Asp Ile Gln Glu Leu Tyr Ile Arg Val Val Asp Lys Val Glu Ile Gly
            980                 985                 990

Lys Thr Val Lys Ala Tyr Val Arg  Val Leu Asp Leu His  Lys Lys Pro
        995                 1000                1005

Phe Leu  Ala Lys Tyr Phe Pro  Phe Met Asp Leu Lys  Leu Arg Ala
    1010                1015                1020

Ala Ser  Pro Ile Ile Thr Leu  Val Ala Leu Asp Glu  Ala Leu Asp
    1025                1030                1035

Asn Tyr  Thr Ile Thr Phe Leu  Ile Arg Gly Val Ala  Ile Gly Gln
    1040                1045                1050

Thr Ser  Leu Thr Ala Ser Val  Thr Asn Lys Ala Gly  Gln Arg Ile
    1055                1060                1065

Asn Ser  Ala Pro Gln Gln Ile  Glu Val Phe Pro Pro  Phe Arg Leu
    1070                1075                1080

Met Pro  Arg Lys Val Thr Leu  Leu Ile Gly Ala Thr  Met Gln Val
    1085                1090                1095

Thr Ser  Glu Gly Gly Pro Gln  Pro Gln Ser Asn Ile  Leu Phe Ser
    1100                1105                1110

Ile Ser Asn Glu Ser Val Ala Leu Val Ser Ala Ala Gly Leu Val
    1115                1120                1125

Gln Gly Leu Ala Ile Gly Asn Gly Thr Val Ser Gly Leu Val Gln
    1130                1135                1140

Ala Val Asp Ala Glu Thr Gly Lys Val Val Ile Ile Ser Gln Asp
    1145                1150                1155

Leu Val Gln Val Glu Val Leu Leu Leu Arg Ala Val Arg Ile Arg
    1160                1165                1170

Ala Pro Ile Met Arg Met Arg Thr Gly Thr Gln Met Pro Ile Tyr
    1175                1180                1185

Val Thr Gly Ile Thr Asn His Gln Asn Pro Phe Ser Phe Gly Asn
    1190                1195                1200

Ala Val Pro Gly Leu Thr Phe His Trp Ser Val Thr Lys Arg Asp
    1205                1210                1215

Val Leu Asp Leu Arg Gly Arg His His Glu Ala Ser Ile Arg Leu
    1220                1225                1230

Pro Ser Gln Tyr Asn Phe Ala Met Asn Val Leu Gly Arg Val Lys
    1235                1240                1245

Gly Arg Thr Gly Leu Arg Val Val Val Lys Ala Val Asp Pro Thr
    1250                1255                1260

Ser Gly Gln Leu Tyr Gly Leu Ala Arg Glu Leu Ser Asp Glu Ile
    1265                1270                1275

Gln Val Gln Val Phe Glu Lys Leu Gln Leu Leu Asn Pro Glu Ile
    1280                1285                1290

Glu Ala Glu Gln Ile Leu Met Ser Pro Asn Ser Tyr Ile Lys Leu
    1295                1300                1305

Gln Thr Asn Arg Asp Gly Ala Ala Ser Leu Ser Tyr Arg Val Leu
    1310                1315                1320

Asp Gly Pro Glu Lys Val Pro Val Val His Val Asp Glu Lys Gly
    1325                1330                1335

Phe Leu Ala Ser Gly Ser Met Ile Gly Thr Ser Thr Ile Glu Val
    1340                1345                1350

Ile Ala Gln Glu Pro Phe Gly Ala Asn Gln Thr Ile Ile Val Ala
    1355                1360                1365

Val Lys Val Ser Pro Val Ser Tyr Leu Arg Val Ser Met Ser Pro
    1370                1375                1380

Val Leu His Thr Gln Asn Lys Glu Ala Leu Val Ala Val Pro Leu
    1385                1390                1395

Gly Met Thr Val Thr Phe Thr Val His Phe His Asp Asn Ser Gly
    1400                1405                1410

Asp Val Phe His Ala His Ser Ser Val Leu Asn Phe Ala Thr Asn
    1415                1420                1425

Arg Asp Asp Phe Val Gln Ile Gly Lys Gly Pro Thr Asn Asn Thr
    1430                1435                1440

Cys Val Val Arg Thr Val Ser Val Gly Leu Thr Leu Leu Arg Val
    1445                1450                1455

Trp Asp Ala Glu His Pro Gly Leu Ser Asp Phe Met Pro Leu Pro
    1460                1465                1470

Val Leu Gln Ala Ile Ser Pro Glu Leu Ser Gly Ala Met Val Val
    1475                1480                1485

Gly Asp Val Leu Cys Leu Ala Thr Val Leu Thr Ser Leu Glu Gly
    1490                1495                1500

Leu Ser Gly Thr Trp Ser Ser Ser Ala Asn Ser Ile Leu His Ile
    1505                1510                1515

Asp Pro Lys Thr Gly Val Ala Val Ala Arg Ala Val Gly Ser Val
    1520                1525                1530

Thr Val Tyr Tyr Glu Val Ala Gly His Leu Arg Thr Tyr Lys Glu
    1535                1540                1545

Val Val Val Ser Val Pro Gln Arg Ile Met Ala Arg His Leu His
    1550                1555                1560

Pro Ile Gln Thr Ser Phe Gln Glu Ala Thr Ala Ser Lys Val Ile
    1565                1570                1575

Val Ala Val Gly Asp Arg Ser Ser Asn Leu Arg Gly Glu Cys Thr
    1580                1585                1590

Pro Thr Gln Arg Glu Val Ile Gln Ala Leu His Pro Glu Thr Leu
    1595                1600                1605

Ile Ser Cys Gln Ser Gln Phe Lys Pro Ala Val Phe Asp Phe Pro
    1610                1615                1620

Ser Gln Asp Val Phe Thr Val Glu Pro Gln Phe Asp Thr Ala Leu
    1625                    1630                    1635

Gly Gln Tyr Phe Cys Ser Ile Thr Met His Arg Leu Thr Asp Lys
    1640                    1645                    1650

Gln Arg Lys His Leu Ser Met Lys Lys Thr Ala Leu Val Val Ser
    1655                    1660                    1665

Ala Ser Leu Ser Ser Ser His Phe Ser Thr Glu Gln Val Gly Ala
    1670                    1675                    1680

Glu Val Pro Phe Ser Pro Gly Leu Phe Ala Asp Gln Ala Glu Ile
    1685                    1690                    1695

Leu Leu Ser Asn His Tyr Thr Ser Ser Glu Ile Arg Val Phe Gly
    1700                    1705                    1710

Ala Pro Glu Val Leu Glu Asn Leu Glu Val Lys Ser Gly Ser Pro
    1715                    1720                    1725

Ala Val Leu Ala Phe Ala Lys Glu Lys Ser Phe Gly Trp Pro Ser
    1730                    1735                    1740

Phe Ile Thr Tyr Thr Val Gly Val Leu Asp Pro Ala Ala Gly Ser
    1745                    1750                    1755

Gln Gly Pro Leu Ser Thr Thr Leu Thr Phe Ser Ser Pro Val Thr
    1760                    1765                    1770

Asn Gln Ala Ile Ala Ile Pro Val Thr Val Ala Phe Val Val Asp
    1775                    1780                    1785

Arg Arg Gly Pro Gly Pro Tyr Gly Ala Ser Leu Phe Gln His Phe
    1790                    1795                    1800

Leu Asp Ser Tyr Gln Val Met Phe Phe Thr Leu Phe Ala Leu Leu
    1805                    1810                    1815

Ala Gly Thr Ala Val Met Ile Ile Ala Tyr His Thr Val Cys Thr
    1820                    1825                    1830

Pro Arg Asp Leu Ala Val Pro Ala Ala Leu Thr Pro Arg Ala Ser
    1835                    1840                    1845

Pro Gly His Ser Pro His Tyr Phe Ala Ala Ser Ser Pro Thr Ser
    1850                    1855                    1860

Pro Asn Ala Leu Pro Pro Ala Arg Lys Ala Ser Pro Pro Ser Gly
    1865                    1870                    1875

Leu Trp  Ser Pro Ala Tyr Ala  Ser His
    1880                1885

<210>  15
<211>  2907
<212>  PRT
<213>  Homo sapiens

<400>  15

Met Val Ser Glu Glu Glu Glu Glu Glu Asp Gly Asp Ala Glu Glu Thr
1               5                   10                  15

Gln Asp Ser Glu Asp Asp Glu Glu Asp Glu Met Glu Glu Asp Asp Asp
            20                  25                  30

Asp Ser Asp Tyr Pro Glu Glu Met Glu Asp Asp Asp Asp Asp Ala Ser
        35                  40                  45

Tyr Cys Thr Glu Ser Ser Phe Arg Ser His Ser Thr Tyr Ser Ser Thr
    50                  55                  60

Pro Gly Arg Arg Lys Pro Arg Val His Arg Pro Arg Ser Pro Ile Leu
65                  70                  75                  80

Glu Glu Lys Asp Ile Pro Pro Leu Glu Phe Pro Lys Ser Ser Glu Asp
                85                  90                  95

Leu Met Val Pro Asn Glu His Ile Met Asn Val Ile Ala Ile Tyr Glu
            100                 105                 110

Val Leu Arg Asn Phe Gly Thr Val Leu Arg Leu Ser Pro Phe Arg Phe
        115                 120                 125

Glu Asp Phe Cys Ala Ala Leu Val Ser Gln Glu Gln Cys Thr Leu Met
    130                 135                 140

Ala Glu Met His Val Val Leu Leu Lys Ala Val Leu Arg Glu Glu Asp
145                 150                 155                 160

Thr Ser Asn Thr Thr Phe Gly Pro Ala Asp Leu Lys Asp Ser Val Asn
                165                 170                 175

Ser Thr Leu Tyr Phe Ile Asp Gly Met Thr Trp Pro Glu Val Leu Arg
            180                 185                 190

Val Tyr Cys Glu Ser Asp Lys Glu Tyr His His Val Leu Pro Tyr Gln
        195                 200                 205

Glu Ala Glu Asp Tyr Pro Tyr Gly Pro Val Glu Asn Lys Ile Lys Val
    210                 215                 220

Leu Gln Phe Leu Val Asp Gln Phe Leu Thr Thr Asn Ile Ala Arg Glu
225                 230                 235                 240

Glu Leu Met Ser Glu Gly Val Ile Gln Tyr Asp Asp His Cys Arg Val
                245                 250                 255

Cys His Lys Leu Gly Asp Leu Leu Cys Cys Glu Thr Cys Ser Ala Val
            260                 265                 270

Tyr His Leu Glu Cys Val Lys Pro Pro Leu Glu Glu Val Pro Glu Asp
        275                 280                 285

Glu Trp Gln Cys Glu Val Cys Val Ala His Lys Val Pro Gly Val Thr
    290                 295                 300

Asp Cys Val Ala Glu Ile Gln Lys Asn Lys Pro Tyr Ile Arg His Glu
305                 310                 315                 320

Pro Ile Gly Tyr Asp Arg Ser Arg Arg Lys Tyr Trp Phe Leu Asn Arg
                325                 330                 335

Arg Leu Ile Ile Glu Glu Asp Thr Glu Asn Glu Asn Glu Lys Lys Ile
            340                 345                 350

Trp Tyr Tyr Ser Thr Lys Val Gln Leu Ala Glu Leu Ile Asp Cys Leu
        355                 360                 365

Asp Lys Asp Tyr Trp Glu Ala Glu Leu Cys Lys Ile Leu Glu Glu Met
    370                 375                 380

Arg Glu Glu Ile His Arg His Met Asp Ile Thr Glu Asp Leu Thr Asn
385                 390                 395                 400

Lys Ala Arg Gly Ser Asn Lys Ser Phe Leu Ala Ala Ala Asn Glu Glu
                405                 410                 415

Ile Leu Glu Ser Ile Arg Ala Lys Lys Gly Asp Ile Asp Asn Val Lys
            420                 425                 430

Ser Pro Glu Glu Thr Glu Lys Asp Lys Asn Glu Thr Glu Asn Asp Ser
        435                 440                 445

Lys Asp Ala Glu Lys Asn Arg Glu Glu Phe Glu Asp Gln Ser Leu Glu
    450                 455                 460

Lys Asp Ser Asp Asp Lys Thr Pro Asp Asp Asp Pro Glu Gln Gly Lys
465                 470                 475                 480

Ser Glu Glu Pro Thr Glu Val Gly Asp Lys Gly Asn Ser Val Ser Ala
                485                 490                 495

Asn Leu Gly Asp Asn Thr Thr Asn Ala Thr Ser Glu Glu Thr Ser Pro
            500                 505                 510

Ser Glu Gly Arg Ser Pro Val Gly Cys Leu Ser Glu Thr Pro Asp Ser
        515                 520                 525

Ser Asn Met Ala Glu Lys Lys Val Ala Ser Glu Leu Pro Gln Asp Val
    530                 535                 540

Pro Glu Glu Pro Asn Lys Thr Cys Glu Ser Ser Asn Thr Ser Ala Thr
545                 550                 555                 560

Thr Thr Ser Ile Gln Pro Asn Leu Glu Asn Ser Asn Ser Ser Ser Glu
                565                 570                 575

Leu Asn Ser Ser Gln Ser Glu Ser Ala Lys Ala Ala Asp Asp Pro Glu
            580                 585                 590

Asn Gly Glu Arg Glu Ser His Thr Pro Val Ser Ile Gln Glu Glu Ile
        595                 600                 605

Val Gly Asp Phe Lys Ser Glu Lys Ser Asn Gly Glu Leu Ser Glu Ser
    610                 615                 620

Pro Gly Ala Gly Lys Gly Ala Ser Gly Ser Thr Arg Ile Ile Thr Arg
625                 630                 635                 640

Leu Arg Asn Pro Asp Ser Lys Leu Ser Gln Leu Lys Ser Gln Gln Val
                645                 650                 655

Ala Ala Ala Ala His Glu Ala Asn Lys Leu Phe Lys Glu Gly Lys Glu
            660                 665                 670

Val Leu Val Val Asn Ser Gln Gly Glu Ile Ser Arg Leu Ser Thr Lys
        675                 680                 685

Lys Glu Val Ile Met Lys Gly Asn Ile Asn Asn Tyr Phe Lys Leu Gly
    690                 695                 700

Gln Glu Gly Lys Tyr Arg Val Tyr His Asn Gln Tyr Ser Thr Asn Ser
705                 710                 715                 720

Phe Ala Leu Asn Lys His Gln His Arg Glu Asp His Asp Lys Arg Arg
                725                 730                 735

His Leu Ala His Lys Phe Cys Leu Thr Pro Ala Gly Glu Phe Lys Trp
            740                 745                 750

Asn Gly Ser Val His Gly Ser Lys Val Leu Thr Ile Ser Thr Leu Arg
        755                 760                 765

Leu Thr Ile Thr Gln Leu Glu Asn Asn Ile Pro Ser Ser Phe Leu His
    770                 775                 780

Pro Asn Trp Ala Ser His Arg Ala Asn Trp Ile Lys Ala Val Gln Met
785                 790                 795                 800

Cys Ser Lys Pro Arg Glu Phe Ala Leu Ala Leu Ala Ile Leu Glu Cys
                805                 810                 815

Ala Val Lys Pro Val Val Met Leu Pro Ile Trp Arg Glu Ser Leu Gly
            820                 825                 830

His Thr Arg Leu His Arg Met Thr Ser Ile Glu Arg Glu Glu Lys Glu
        835                 840                 845

Lys Val Lys Lys Lys Glu Lys Lys Gln Glu Glu Glu Glu Thr Met Gln
    850                 855                 860

Gln Ala Thr Trp Val Lys Tyr Thr Phe Pro Val Lys His Gln Val Trp
865                 870                 875                 880

Lys Gln Lys Gly Glu Glu Tyr Arg Val Thr Gly Tyr Gly Gly Trp Ser
                885                 890                 895

Trp Ile Ser Lys Thr His Val Tyr Arg Phe Val Pro Lys Leu Pro Gly
            900                 905                 910

Asn Thr Asn Val Asn Tyr Arg Lys Ser Leu Glu Gly Thr Lys Asn Asn
        915                 920                 925

Met Asp Glu Asn Met Asp Glu Ser Asp Lys Arg Lys Cys Ser Arg Ser
    930                 935                 940

Pro Lys Lys Ile Lys Ile Glu Pro Asp Ser Glu Lys Asp Glu Val Lys
945                 950                 955                 960

Gly Ser Asp Ala Ala Lys Gly Ala Asp Gln Asn Glu Met Asp Ile Ser
                965                 970                 975

Lys Ile Thr Glu Lys Lys Asp Gln Asp Val Lys Glu Leu Leu Asp Ser
            980                 985                 990

Asp Ser Asp Lys Pro Cys Lys Glu  Glu Pro Met Glu Val  Asp Asp Asp
        995                 1000                1005

Met Lys  Thr Glu Ser His Val  Asn Cys Gln Glu Ser  Ser Gln Val
    1010                1015                1020

Asp Val  Val Asn Val Ser Glu  Gly Phe His Leu Arg  Thr Ser Tyr
    1025                1030                1035

Lys Lys Lys Thr Lys Ser Ser Lys Leu Asp Gly Leu Leu Glu Arg
    1040                1045                1050

Arg Ile Lys Gln Phe Thr Leu Glu Glu Lys Gln Arg Leu Glu Lys
    1055                1060                1065

Ile Lys Leu Glu Gly Gly Ile Lys Gly Ile Gly Lys Thr Ser Thr
    1070                1075                1080

Asn Ser Ser Lys Asn Leu Ser Glu Ser Pro Val Ile Thr Lys Ala
    1085                1090                1095

Lys Glu Gly Cys Gln Ser Asp Ser Met Arg Gln Glu Gln Ser Pro
    1100                1105                1110

Asn Ala Asn Asn Asp Gln Pro Glu Asp Leu Ile Gln Gly Cys Ser
    1115                1120                1125

Glu Ser Asp Ser Ser Val Leu Arg Met Ser Asp Pro Ser His Thr
    1130                1135                1140

Thr Asn Lys Leu Tyr Pro Lys Asp Arg Val Leu Asp Asp Val Ser
    1145                1150                1155

Ile Arg Ser Pro Glu Thr Lys Cys Pro Lys Gln Asn Ser Ile Glu
    1160                1165                1170

Asn Asp Ile Glu Glu Lys Val Ser Asp Leu Ala Ser Arg Gly Gln
    1175                1180                1185

Glu Pro Ser Lys Ser Lys Thr Lys Gly Asn Asp Phe Phe Ile Asp
    1190                1195                1200

Asp Ser Lys Leu Ala Ser Ala Asp Asp Ile Gly Thr Leu Ile Cys
    1205                1210                1215

Lys Asn Lys Lys Pro Leu Ile Gln Glu Glu Ser Asp Thr Ile Val
    1220                1225                1230

Ser Ser Ser Lys Ser Ala Leu His Ser Ser Val Pro Lys Ser Thr
    1235                1240                1245

Asn Asp Arg Asp Ala Thr Pro Leu Ser Arg Ala Met Asp Phe Glu
    1250                1255                1260

Gly Lys Leu Gly Cys Asp Ser Glu Ser Asn Ser Thr Leu Glu Asn
    1265                1270                1275

Ser Ser Asp Thr Val Ser Ile Gln Asp Ser Ser Glu Glu Asp Met
    1280                1285                1290

Ile Val Gln Asn Ser Asn Glu Ser Ile Ser Glu Gln Phe Arg Thr
    1295                1300                1305

Arg Glu Gln Asp Val Glu Val Leu Glu Pro Leu Lys Cys Glu Leu
    1310                1315                1320

Val Ser Gly Glu Ser Thr Gly Asn Cys Glu Asp Arg Leu Pro Val
    1325                1330                1335

Lys Gly Thr Glu Ala Asn Gly Lys Lys Pro Ser Gln Gln Lys Lys
    1340                1345                1350

Leu Glu Glu Arg Pro Val Asn Lys Cys Ser Asp Gln Ile Lys Leu
    1355                1360                1365

Lys Asn Thr Thr Asp Lys Lys Asn Asn Glu Asn Arg Glu Ser Glu
    1370                1375                1380

Lys Lys Gly Gln Arg Thr Ser Thr Phe Gln Ile Asn Gly Lys Asp
    1385                1390                1395

Asn Lys Pro Lys Ile Tyr Leu Lys Gly Glu Cys Leu Lys Glu Ile
    1400                1405                1410

Ser Glu Ser Arg Val Val Ser Gly Asn Val Glu Pro Lys Val Asn
    1415                1420                1425

Asn Ile Asn Lys Ile Ile Pro Glu Asn Asp Ile Lys Ser Leu Thr
    1430                1435                1440

Val Lys Glu Ser Ala Ile Arg Pro Phe Ile Asn Gly Asp Val Ile
    1445                1450                1455

Met Glu Asp Phe Asn Glu Arg Asn Ser Ser Glu Thr Lys Ser His
    1460                1465                1470

Leu Leu Ser Ser Ser Asp Ala Glu Gly Asn Tyr Arg Asp Ser Leu
    1475                1480                1485

Glu Thr Leu Pro Ser Thr Lys Glu Ser Asp Ser Thr Gln Thr Thr
    1490                1495                1500

Thr Pro Ser Ala Ser Cys Pro Glu Ser Asn Ser Val Asn Gln Val
    1505                1510                1515

Glu Asp Met Glu Ile Glu Thr Ser Glu Val Lys Lys Val Thr Ser
    1520                1525                1530

Ser Pro Ile Thr Ser Glu Glu Glu Ser Asn Leu Ser Asn Asp Phe
    1535                1540                1545

Ile Asp Glu Asn Gly Leu Pro Ile Asn Lys Asn Glu Asn Val Asn
    1550                1555                1560

Gly Glu Ser Lys Arg Lys Thr Val Ile Thr Glu Val Thr Thr Met
    1565                1570                1575

Thr Ser Thr Val Ala Thr Glu Ser Lys Thr Val Ile Lys Val Glu
    1580                1585                1590

Lys Gly Asp Lys Gln Thr Val Val Ser Ser Thr Glu Asn Cys Ala
    1595                1600                1605

Lys Ser Thr Val Thr Thr Thr Thr Thr Thr Val Thr Lys Leu Ser
    1610                1615                1620

Thr Pro Ser Thr Gly Gly Ser Val Asp Ile Ile Ser Val Lys Glu
    1625                1630                1635

Gln Ser Lys Thr Val Val Thr Thr Thr Val Thr Asp Ser Leu Thr
    1640                1645                1650

Thr Thr Gly Gly Thr Leu Val Thr Ser Met Thr Val Ser Lys Glu
    1655                1660                1665

Tyr Ser Thr Arg Asp Lys Val Lys Leu Met Lys Phe Ser Arg Pro
    1670                1675                1680

Lys Lys Thr Arg Ser Gly Thr Ala Leu Pro Ser Tyr Arg Lys Phe
    1685                1690                1695

Val Thr Lys Ser Thr Lys Lys Ser Ile Phe Val Leu Pro Asn Asp
    1700                1705                1710

Asp Leu Lys Lys Leu Ala Arg Lys Gly Gly Ile Arg Glu Val Pro
    1715                1720                1725

Tyr Phe Asn Tyr Asn Ala Lys Pro Ala Leu Asp Ile Trp Pro Tyr
    1730                1735                1740

Pro Ser Pro Arg Pro Thr Phe Gly Ile Thr Trp Arg Tyr Arg Leu
    1745                1750                1755

Gln Thr Val Lys Ser Leu Ala Gly Val Ser Leu Met Leu Arg Leu
    1760                1765                1770

Leu Trp Ala Ser Leu Arg Trp Asp Asp Met Ala Ala Lys Val Pro
    1775                1780                1785

Pro Gly Gly Gly Ser Thr Arg Thr Glu Thr Ser Glu Thr Glu Ile
    1790                1795                1800

Thr Thr Thr Glu Ile Ile Lys Arg Arg Asp Val Gly Pro Tyr Gly
    1805                1810                1815

Ile Arg Phe Glu Tyr Cys Ile Arg Lys Ile Ile Cys Pro Ile Gly
    1820                1825                1830

Val Pro Glu Thr Pro Lys Glu Thr Pro Thr Pro Gln Arg Lys Gly
    1835                1840                1845

Leu Arg Ser Ser Ala Leu Arg Pro Lys Arg Pro Glu Thr Pro Lys
    1850                1855                1860

Gln Thr Gly Pro Val Ile Ile Glu Thr Trp Val Ala Glu Glu Glu
    1865                1870                1875

Leu Glu Leu Trp Glu Ile Arg Ala Phe Ala Glu Arg Val Glu Lys
    1880                1885                1890

Glu Lys Ala Gln Ala Val Glu Gln Gln Ala Lys Lys Arg Leu Glu
    1895                1900                1905

Gln Gln Lys Pro Thr Val Ile Ala Thr Ser Thr Thr Ser Pro Thr
    1910                1915                1920

Ser Ser Thr Thr Ser Thr Ile Ser Pro Ala Gln Lys Val Met Val
    1925                1930                1935

Ala Pro Ile Ser Gly Ser Val Thr Thr Gly Thr Lys Met Val Leu
    1940                1945                1950

Thr Thr Lys Val Gly Ser Pro Ala Thr Val Thr Phe Gln Gln Asn
    1955                1960                1965

Lys Asn Phe His Gln Thr Phe Ala Thr Trp Val Lys Gln Gly Gln
    1970                1975                1980

Ser Asn Ser Gly Val Val Gln Val Gln Gln Lys Val Leu Gly Ile
    1985                1990                1995

Ile Pro Ser Ser Thr Gly Thr Ser Gln Gln Thr Phe Thr Ser Phe
    2000                2005                2010

Gln Pro Arg Thr Ala Thr Val Thr Ile Arg Pro Asn Thr Ser Gly
    2015                2020                2025

Ser Gly Gly Thr Thr Ser Asn Ser Gln Val Ile Thr Gly Pro Gln
    2030                2035                2040

Ile Arg Pro Gly Met Thr Val Ile Arg Thr Pro Leu Gln Gln Ser
    2045                2050                2055

Thr Leu Gly Lys Ala Ile Ile Arg Thr Pro Val Met Val Gln Pro
    2060                2065                2070

Gly Ala Pro Gln Gln Val Met Thr Gln Ile Ile Arg Gly Gln Pro
    2075                2080                2085

Val Ser Thr Ala Val Ser Ala Pro Asn Thr Val Ser Ser Thr Pro
    2090                2095                2100

Gly Gln Lys Ser Leu Thr Ser Ala Thr Ser Thr Ser Asn Ile Gln
    2105                2110                2115

Ser Ser Ala Ser Gln Pro Pro Arg Pro Gln Gln Gly Gln Val Lys
    2120                2125                2130

Leu Thr Met Ala Gln Leu Thr Gln Leu Thr Gln Gly His Gly Gly
    2135                2140                2145

Asn Gln Gly Leu Thr Val Val Ile Gln Gly Gln Gly Gln Thr Thr
    2150                2155                2160

Gly Gln Leu Gln Leu Ile Pro Gln Gly Val Thr Val Leu Pro Gly
    2165                2170                2175

Pro Gly Gln Gln Leu Met Gln Ala Ala Met Pro Asn Gly Thr Val
    2180                2185                2190

Gln Arg Phe Leu Phe Thr Pro Leu Ala Thr Thr Ala Thr Thr Ala
    2195                2200                2205

Ser Thr Thr Thr Thr Thr Val Ser Thr Thr Ala Ala Gly Thr Gly
    2210                2215                2220

Glu Gln Arg Gln Ser Lys Leu Ser Pro Gln Met Gln Val His Gln
    2225                2230                2235

Asp Lys Thr Leu Pro Pro Ala Gln Ser Ser Ser Val Gly Pro Ala
    2240                2245                2250

Lys Ala Gln Pro Gln Thr Ala Gln Pro Ser Ala Arg Pro Gln Pro
    2255                2260                2265

Gln Thr Gln Pro Gln Ser Pro Ala Gln Pro Glu Val Gln Thr Gln
    2270                2275                2280

Pro Glu Val Gln Thr Gln Thr Thr Val Ser Ser His Val Pro Ser
    2285                2290                2295

Glu Ala Gln Pro Thr His Ala Gln Ser Ser Lys Pro Gln Val Ala
    2300                2305                2310

Ala Gln Ser Gln Pro Gln Ser Asn Val Gln Gly Gln Ser Pro Val
    2315                2320                2325

Arg Val Gln Ser Pro Ser Gln Thr Arg Ile Arg Pro Ser Thr Pro
    2330                2335                2340

Ser Gln Leu Ser Pro Gly Gln Gln Ser Gln Val Gln Thr Thr Thr
    2345                2350                2355

Ser Gln Pro Ile Pro Ile Gln Pro His Thr Ser Leu Gln Ile Pro
    2360                2365                2370

Ser Gln Gly Gln Pro Gln Ser Gln Pro Gln Val Gln Ser Ser Thr
    2375                2380                2385

Gln Thr Leu Ser Ser Gly Gln Thr Leu Asn Gln Val Ser Val Ser
    2390                2395                2400

Ser Pro Ser Arg Pro Gln Leu Gln Ile Gln Gln Pro Gln Pro Gln
    2405                2410                2415

Val Ile Ala Val Pro Gln Leu Gln Gln Gln Val Gln Val Leu Ser
    2420                2425                2430

Gln Ile Gln Ser Gln Val Val Ala Gln Ile Gln Ala Gln Gln Ser
    2435                2440                2445

Gly Val Pro Gln Gln Ile Lys Leu Gln Leu Pro Ile Gln Ile Gln
    2450                2455                2460

Gln Ser Ser Ala Val Gln Thr His Gln Ile Gln Asn Val Val Thr
    2465                2470                2475

Val Gln Ala Ala Ser Val Gln Glu Gln Leu Gln Arg Val Gln Gln
    2480                2485                2490

Leu Arg Asp Gln Gln Gln Lys Lys Lys Gln Gln Gln Ile Glu Ile
    2495                2500                2505

Lys Arg Glu His Thr Leu Gln Ala Ser Asn Gln Ser Glu Ile Ile
    2510                2515                2520

Gln Lys Gln Val Val Met Lys His Asn Ala Val Ile Glu His Leu
    2525                2530                2535

Lys Gln Lys Lys Ser Met Thr Pro Ala Glu Arg Glu Glu Asn Gln
    2540                2545                2550

Arg Met Ile Val Cys Asn Gln Val Met Lys Tyr Ile Leu Asp Lys
    2555                2560                2565

Ile Asp Lys Glu Glu Lys Gln Ala Ala Lys Lys Arg Lys Arg Glu
    2570                2575                2580

Glu Ser Val Glu Gln Lys Arg Ser Lys Gln Asn Ala Thr Lys Leu
    2585                2590                2595

Ser Ala Leu Leu Phe Lys His Lys Glu Gln Leu Arg Ala Glu Ile
    2600                2605                2610

Leu Lys Lys Arg Ala Leu Leu Asp Lys Asp Leu Gln Ile Glu Val
    2615                2620                2625

Gln Glu Glu Leu Lys Arg Asp Leu Lys Ile Lys Lys Glu Lys Asp
    2630                2635                2640

Leu Met Gln Leu Ala Gln Ala Thr Ala Val Ala Ala Pro Cys Pro
    2645                2650                2655

Pro Val Thr Pro Ala Pro Pro Ala Pro Pro Ala Pro Pro Pro Ser
    2660                2665                2670

Pro Pro Pro Pro Pro Ala Val Gln His Thr Gly Leu Leu Ser Thr
    2675                2680                2685

Pro Thr Leu Pro Ala Ala Ser Gln Lys Arg Lys Arg Glu Glu Glu
    2690                2695                2700

Lys Asp Ser Ser Ser Lys Ser Lys Lys Lys Lys Met Ile Ser Thr
    2705                2710                2715

Thr Ser Lys Glu Thr Lys Lys Asp Thr Lys Leu Tyr Cys Ile Cys
    2720                2725                2730

Lys Thr Pro Tyr Asp Glu Ser Lys Phe Tyr Ile Gly Cys Asp Arg
    2735                2740                2745

Cys Gln Asn Trp Tyr His Gly Arg Cys Val Gly Ile Leu Gln Ser
    2750                2755                2760

Glu Ala Glu Leu Ile Asp Glu Tyr Val Cys Pro Gln Cys Gln Ser
    2765                2770                2775

Thr Glu Asp Ala Met Thr Val Leu Thr Pro Leu Thr Glu Lys Asp
    2780                2785                2790

Tyr Glu Gly Leu Lys Arg Val Leu Arg Ser Leu Gln Ala His Lys
    2795                2800                2805

Met Ala Trp Pro Phe Leu Glu Pro Val Asp Pro Asn Asp Ala Pro
    2810                2815                2820

Asp Tyr Tyr Gly Val Ile Lys Glu Pro Met Asp Leu Ala Thr Met
    2825                2830                2835

Glu Glu Arg Val Gln Arg Arg Tyr Tyr Glu Lys Leu Thr Glu Phe
    2840                2845                2850

Val Ala Asp Met Thr Lys Ile Phe Asp Asn Cys Arg Tyr Tyr Asn
    2855                2860                2865

Pro Ser Asp Ser Pro Phe Tyr Gln Cys Ala Glu Val Leu Glu Ser
    2870                2875                2880

Phe Phe Val Gln Lys Leu Lys Gly Phe Lys Ala Ser Arg Ser His
    2885                2890                2895

Asn Asn Lys Leu Gln Ser Thr Ala Ser
    2900                2905

<210> 16
<211> 110
<212> PRT
<213> Homo sapiens

<400> 16

Met Arg Phe Met Thr Leu Leu Phe Leu Thr Ala Leu Ala Gly Ala Leu
1               5                   10                  15

Val Cys Ala Tyr Asp Pro Glu Ala Ala Ser Ala Pro Gly Ser Gly Asn
            20                  25                  30

Pro Cys His Glu Ala Ser Ala Ala Gln Lys Glu Asn Ala Gly Glu Asp
        35                  40                  45

Pro Gly Leu Ala Arg Gln Ala Pro Lys Pro Arg Lys Gln Arg Ser Ser
    50                  55                  60

Leu Leu Glu Lys Gly Leu Asp Gly Ala Lys Lys Ala Val Gly Gly Leu
65                  70                  75                  80

Gly Lys Leu Gly Lys Asp Ala Val Glu Asp Leu Glu Ser Val Gly Lys
                85                  90                  95

Gly Ala Val His Asp Val Lys Asp Val Leu Asp Ser Val Leu
            100                 105                 110

<210> 17
<211> 109
<212> PRT
<213> Homo sapiens

<400> 17

<210>  18
<211>  166
<212>  PRT
<213>  Homo sapiens

<400>  18

Met Ala Ser Gly Val Ala Val Ser Asp Gly Val Ile Lys Val Phe Asn
1               5                   10                  15

Asp Met Lys Val Arg Lys Ser Ser Thr Pro Glu Glu Val Lys Lys Arg
            20                  25                  30

Lys Lys Ala Val Leu Phe Cys Leu Ser Glu Asp Lys Lys Asn Ile Ile
        35                  40                  45

Leu Glu Glu Gly Lys Glu Ile Leu Val Gly Asp Val Gly Gln Thr Val
    50                  55                  60

Asp Asp Pro Tyr Ala Thr Phe Val Lys Met Leu Pro Asp Lys Asp Cys
65                  70                  75                  80

Arg Tyr Ala Leu Tyr Asp Ala Thr Tyr Glu Thr Lys Glu Ser Lys Lys
                85                  90                  95

Glu Asp Leu Val Phe Ile Phe Trp Ala Pro Glu Ser Ala Pro Leu Lys
            100                 105                 110

Ser Lys Met Ile Tyr Ala Ser Ser Lys Asp Ala Ile Lys Lys Lys Leu
        115                 120                 125

Thr Gly Ile Lys His Glu Leu Gln Ala Asn Cys Tyr Glu Glu Val Lys
    130                 135                 140

Asp Arg Cys Thr Leu Ala Glu Lys Leu Gly Gly Ser Ala Val Ile Ser
145                 150                 155                 160

Leu Glu Gly Lys Pro Leu
                165

<210>  19
<211>  134
<212>  PRT
<213>  Homo sapiens

<400>  19

Met Val Leu Gln Thr Gln Val Phe Ile Ser Leu Leu Leu Trp Ile Ser
1               5                   10                  15

Gly Ala Tyr Gly Asp Ile Val Met Thr Gln Ser Pro Asp Ser Leu Ala
            20                  25                  30

Val Ser Leu Gly Glu Arg Ala Thr Ile Asn Cys Lys Ser Ser Gln Ser
        35                  40                  45

Ile Leu Tyr Ser Ser Asp Asn Lys Asn Tyr Leu Ala Trp Tyr Gln Gln
    50                  55                  60

Lys Pro Gly Gln Pro Pro Lys Leu Leu Ile Tyr Trp Ala Ser Thr Arg
65                  70                  75                  80

Glu Ser Gly Val Pro Asp Arg Phe Ser Gly Ser Gly Ser Gly Thr Asp
                85                  90                  95

Phe Thr Leu Thr Ile Ser Ser Leu Gln Ala Glu Asp Val Ala Val Tyr
            100                 105                 110

Tyr Cys Gln Gln Tyr Tyr Asn Leu Pro Trp Thr Phe Gly Gln Gly Thr
        115                 120                 125

Lys Val Glu Ile Lys Arg
    130

<210>  20
<211>  219
<212>  PRT
<213>  Homo sapiens

<400>  20

Met Ser Glu Thr Ala Pro Ala Ala Pro Ala Ala Pro Ala Pro Ala Glu
1               5                   10                  15

Lys Thr Pro Val Lys Lys Lys Ala Arg Lys Ser Ala Gly Ala Ala Lys
            20                  25                  30

Arg Lys Ala Ser Gly Pro Pro Val Ser Glu Leu Ile Thr Lys Ala Val

35                  40                  45

Ala Ala Ser Lys Glu Arg Ser Gly Val Ser Leu Ala Ala Leu Lys Lys
    50                  55                  60

Ala Leu Ala Ala Ala Gly Tyr Asp Val Glu Lys Asn Asn Ser Arg Ile
65                  70                  75                  80

Lys Leu Gly Leu Lys Ser Leu Val Ser Lys Gly Thr Leu Val Gln Thr
                85                  90                  95

Lys Gly Thr Gly Ala Ser Gly Ser Phe Lys Leu Asn Lys Lys Ala Ala
            100                 105                 110

Ser Gly Glu Ala Lys Pro Lys Ala Lys Lys Ala Gly Ala Ala Lys Ala
        115                 120                 125

Lys Lys Pro Ala Gly Ala Ala Lys Lys Pro Lys Lys Ala Thr Gly Ala
    130                 135                 140

Ala Thr Pro Lys Lys Ser Ala Lys Lys Thr Pro Lys Lys Ala Lys Lys
145                 150                 155                 160

Pro Ala Ala Ala Ala Gly Ala Lys Lys Ala Lys Ser Pro Lys Lys Ala
                165                 170                 175

Lys Ala Ala Lys Pro Lys Lys Ala Pro Lys Ser Pro Ala Lys Ala Lys
            180                 185                 190

Ala Val Lys Pro Lys Ala Ala Lys Pro Lys Thr Ala Lys Pro Lys Ala
        195                 200                 205

Ala Lys Pro Lys Lys Ala Ala Ala Lys Lys Lys
    210                 215

<210>  21
<211>  674
<212>  PRT
<213>  Homo sapiens

<400>  21

Met Ser Ser Ser Ser Arg Gly Pro Gly Ala Gly Ala Arg Arg Arg Arg
1               5                   10                  15

Thr Arg Cys Arg Arg Cys Arg Ala Cys Val Arg Thr Glu Cys Gly Asp
            20                  25                  30

Cys His Phe Cys Arg Asp Met Lys Lys Phe Gly Gly Pro Gly Arg Met
        35                  40                  45

Lys Gln Ser Cys Leu Leu Arg Gln Cys Thr Ala Pro Val Leu Pro His
    50                  55                  60

Thr Ala Val Cys Leu Leu Cys Gly Glu Ala Gly Lys Glu Asp Thr Val
65                  70                  75                  80

Glu Gly Glu Glu Glu Lys Phe Gly Leu Ser Leu Met Glu Cys Thr Ile
                85                  90                  95

Cys Asn Glu Ile Val His Pro Gly Cys Leu Lys Met Gly Lys Ala Glu
            100                 105                 110

Gly Val Ile Asn Ala Glu Ile Pro Asn Cys Trp Glu Cys Pro Arg Cys
        115                 120                 125

Thr Gln Glu Gly Arg Thr Ser Lys Asp Ser Gly Glu Gly Pro Gly Arg
    130                 135                 140

Arg Arg Ala Asp Asn Gly Glu Glu Gly Ala Ser Leu Gly Ser Gly Trp
145                 150                 155                 160

Lys Leu Thr Glu Glu Pro Pro Leu Pro Pro Pro Pro Pro Arg Arg Lys
                165                 170                 175

Gly Pro Leu Pro Ala Gly Pro Pro Pro Glu Asp Val Pro Gly Pro Pro
            180                 185                 190

Lys Arg Lys Glu Arg Glu Ala Gly Asn Glu Pro Pro Thr Pro Arg Lys
        195                 200                 205

Lys Val Lys Gly Gly Arg Glu Arg His Leu Lys Lys Val Gly Gly Asp
    210                 215                 220

Ala Cys Leu Leu Arg Gly Ser Asp Pro Gly Gly Pro Gly Leu Leu Pro
225                 230                 235                 240

Pro Arg Val Leu Asn Pro Ser Gln Ala Phe Ser Ser Cys His Pro Gly
                245                 250                 255

Leu Pro Pro Glu Asn Trp Glu Lys Pro Lys Pro Pro Leu Ala Ser Ala
            260                 265                 270

Glu Gly Pro Ala Val Pro Ser Pro Ser Pro Gln Arg Glu Lys Leu Glu
        275                 280                 285

Arg Phe Lys Arg Met Cys Gln Leu Leu Glu Arg Val Pro Asp Thr Ser
    290                 295                 300

Ser Ser Ser Ser Asp Ser Asp Ser Asp Ser Asp Ser Ser Gly Thr Ser
305                 310                 315                 320

Leu Ser Glu Asp Glu Ala Pro Gly Glu Ala Arg Asn Gly Arg Arg Pro
                325                 330                 335

Ala Arg Gly Ser Ser Gly Glu Lys Glu Asn Arg Gly Gly Arg Arg Ala
            340                 345                 350

Val Arg Pro Gly Ser Gly Gly Pro Leu Leu Ser Trp Pro Leu Gly Pro
        355                 360                 365

Ala Pro Pro Pro Arg Pro Pro Gln Leu Glu Arg His Val Val Arg Pro
    370                 375                 380

Pro Pro Arg Ser Pro Glu Pro Asp Thr Leu Pro Leu Ala Ala Gly Ser
385                 390                 395                 400

Asp His Pro Leu Pro Arg Ala Ala Trp Leu Arg Val Phe Gln His Leu
                405                 410                 415

Gly Pro Arg Glu Leu Cys Ile Cys Met Arg Val Cys Arg Thr Trp Ser
            420                 425                 430

Arg Trp Cys Tyr Asp Lys Arg Leu Trp Pro Arg Met Asp Leu Ser Arg
        435                 440                 445

Arg Lys Ser Leu Thr Pro Pro Met Leu Ser Gly Val Val Arg Arg Gln
    450                 455                 460

Pro Arg Ala Leu Asp Leu Ser Trp Thr Gly Val Ser Lys Lys Gln Leu
465                 470                 475                 480

Met Trp Leu Leu Asn Arg Leu Gln Gly Leu Gln Glu Leu Val Leu Ser
                485                 490                 495

Gly Cys Ser Trp Leu Ser Val Ser Ala Leu Gly Ser Ala Pro Leu Pro
            500                 505                 510

Ala Leu Arg Leu Leu Asp Leu Arg Trp Ile Glu Asp Val Lys Asp Ser
        515                 520                 525

Gln Leu Arg Glu Leu Leu Leu Pro Pro Pro Asp Thr Lys Pro Gly Gln
    530                 535                 540

Thr Glu Ser Arg Gly Arg Leu Gln Gly Val Ala Glu Leu Arg Leu Ala
545                 550                 555                 560

Gly Leu Glu Leu Thr Asp Ala Ser Leu Arg Leu Leu Leu Arg His Ala
                565                 570                 575

Pro Gln Leu Ser Ala Leu Asp Leu Ser His Cys Ala His Val Gly Asp
            580                 585                 590

Pro Ser Val His Leu Leu Thr Ala Pro Thr Ser Pro Leu Arg Glu Thr
        595                 600                 605

Leu Val His Leu Asn Leu Ala Gly Cys His Arg Leu Thr Asp His Cys
    610                 615                 620

Leu Pro Leu Phe Arg Arg Cys Pro Arg Leu Arg Arg Leu Asp Leu Arg
625                 630                 635                 640

Ser Cys Arg Gln Leu Ser Pro Glu Ala Cys Ala Arg Leu Ala Ala Ala
                645                 650                 655

Gly Pro Pro Gly Pro Phe Arg Cys Pro Glu Glu Lys Leu Leu Leu Lys
            660                 665                 670

Asp Ser